# EUROPEAN PATENT SPECIFICATION

(11) **EP 4 359 039 B1**
(45) Date of publication and mention of the grant of the patent: **05.08.2026**
(21) Application number: 22744845.3
(22) Date of filing: 24.06.2022
(51) Int. Cl.: A61M 5/28, A61M 5/50

(54) **CONTAINER FOR EJECTING A SUBSTANCE AND RELATED PRODUCTION METHOD**
BEHÄLTER ZUM AUSSTOSSEN EINER SUBSTANZ UND ZUGEHÖRIGES HERSTELLUNGSVERFAHREN
RÉCIPIENT SERVANT À ÉJECTER UNE SUBSTANCE ET PROCÉDÉ DE PRODUCTION ASSOCIÉ

(30) Priority: 24.06.2021 IT 202100016637
(43) Date of publication of application: 01.05.2024
(73) Proprietor: 3CK S.R.L., 36071 Arzignano (VI) (IT)
(72) Inventor: CONSOLARO, Angelo, 36071 ARZIGNANO (VI) (IT); CONSOLARO, Roberto, 36071 ARZIGNANO (VI) (IT); KABBUR, Rajeev, 36071 ARZIGNANO (VI) (IT); KABBUR, Ajay, 36071 ARZIGNANO (VI) (IT); CONSOLARO, Edoardo, 36071 ARZIGNANO (VI) (IT); CONSOLARO, Francesco Federico, 36071 ARZIGNANO (VI) (IT); CONSOLARO, Massimo, 36071 ARZIGNANO (VI) (IT)
(74) Representative: De Filippis, Sara
(86) International application number: PCT/IT2022/050180
(87) International publication number: WO 2022/269651

(56) References cited:
- GB-A- 1 121 749
- US-A- 4 236 516
- US-A- 4 312 344

## Description

The present invention relates to a container for ejecting at least one substance and the related production method.

### Field of the invention

In more detail, the invention relates to a container for ejecting a substance, the structure of which is such as to maintain the trade-off of integrity between the parts of the container and the "break-loose force" for the functioning of the container, further, the method of use and production thereof is such as to enable the creation of products with different uses and purposes operating under the same principle according to the present invention.

### Known art

As is well known, especially in the medical sector, devices for dispensing substances such as, by way of example only, conventional syringes and pre-filled syringes are widely used.

Generally, a conventional syringe consists of a cylinder containing the substance to be dispensed, a plunger to create pressure to push the liquid and a plunger to push the substance out of the cylinder, and an opening or cannula to allow dispensing. The performance of syringes with a plunger depends on a good compromise between the trigger force and the hydraulic seal between the plunger and the cylinder. In particular, by way of example, this type of seal is designed not to allow any substance or gas to pass between the plunger and the cylinder; furthermore, the seal itself is affected by the interference between the diameters of the cylinder and those of the plunger.

In detail, an initial problem with the above-mentioned type of syringe is the fact that a greater interference between the piston diameter and the cylinder diameter results, on the one hand, in a better integrity of the syringe, but on the other, in an increase in the trigger force and movement of the syringe. For this reason, in all types of syringes, whether made using the 'Blow Fill Seal' (BFS) procedure, or otherwise, lubricants such as silicone oils are used to reduce the friction force and improve the seal of the piston with the cylinder in the syringe.

In particular, the performance of the syringe depends, primarily, on the coefficient of friction between the two elements: piston-cylinder, the amount of lubricant (e.g. silicone oil) present on the piston and/or the lubricant applied to the internal surface of the cylinder.

A further problem lies in the fact that, for example, in a pre-filled syringe, the lubricant that remains in contact with the substance contained in the cylinder, especially when said substance is a drug, could potentially cause aggregates that are highly undesirable because they could contaminate and thus reduce the efficacy of the drug itself.

Nowadays, in an attempt to overcome the aforementioned problems, what is done is to seek a compromise between the amount of lubricant used and the interference surface area between the cylinder and the piston, or to produce pistons and/or cylinders coated with an additional material, with the aim of reducing friction and contamination. However, in the latter case, in order to place the coating on the piston and/or cylinder, it is necessary to introduce additional steps in the production process, which increases costs; furthermore, the new coating material, coming into contact with the substance contained in the cylinder, may also cause contamination and/or undesirable effects.

Not the least of the known state of the art problems is that, for example, in a pre-filled syringe with piston and cylinder, it is important that the ovality of the cylinder diameter is contained within certain limits. If the ovality of the cylinder is greater than these limits, then the piston-cylinder interference must be increased; similarly, the manufacturing process of the cylinder involves a certain tapering of the cylinder which causes problems with piston-cylinder interference. In detail, this step to reduce the ovality and taper of the cylinder leads, disadvantageously, to a more expensive manufacturing process.

Finally, even a traditional cylinder-piston syringe, if subjected to temperature differences and/or excessive pressure, could also be more likely to lose integrity between the piston and cylinder.

At present, there is no container on the market for the ejection of a substance that is economical but, at the same time, allows a compromise between the integrity of the system and the trigger force.

Furthermore, there are currently no known mechanisms in the industry that also guarantee a delivery of substance in the correct quantity.

### Scope of the invention

In the context of the above-mentioned requirements, therefore, the main scope of the present invention is to propose a container for the ejection of a substance, for example of one or more certain medicaments, capable of obviating the technical drawbacks mentioned above and, in particular, to realise a container which guarantees safety in use, whatever its form.

A further object of the present invention is to realise a container for ejecting at least one substance, which guarantees a compromise between the trigger force and the integrity of the system, irrespective of possible variations in temperature and/or pressure.

An additional object of the present invention is to make a container for ejecting at least one substance which is such as to ensure complete delivery of the substance and, if necessary, to ensure non-reusability by the user himself or by third parties.

A further object of the present invention is to realise a container for the ejection of a substance which allows safe use in several respects: hygiene, precision in drug delivery and intrinsic safety for the patient and the operator.

Further, it is an object of the present invention to provide a container, which can be used for making conventional, pre-filled syringes, vials, or other medical or non-medical containers.

It is further an object of the present invention to make a container which is functional, of rapid implementation and sufficiently precise in releasing the medication.

The object of the present invention is thus to create a product which is cost-effective, easy to manufacture, useable and safe.

These and other purposes are achieved by a container for ejecting a substance according to the invention, as will become clear later in this description.

### Object of the invention

It is therefore an object of the present invention to obtain a container for ejecting a substance, such as medicaments and the like, according to claim 1.

In particular, the container object of the present invention comprises a hollow shell for the containment and ejection of the substance and including a containment portion with a dispensing organ of oblong shape, to facilitate the ejection of the above-mentioned substance; moreover, the container also comprises a membrane, connected to the same shell.

Advantageously, the container comprises, in a position contiguous to and transverse with respect to the containment portion, a shaping, for connecting the membrane to the shell.

In an advantageous way, said membrane connected to the shaping of the shell is so that it can move from a first operating position, in which the membrane has a central portion in a position opposite to the containment portion of the shell, so as to create, with the containment portion, a chamber for the containment of the substance, to a second operating position, wherein the central portion of the membrane collapses within the portion of containment of the shell, so as to eject the substance present in the chamber through the dispensing organ.

Additionally, the membrane is calibrated with the geometry of the containment portion of the shell, so as to adhere to and be compressible in the direction of same shell, for ejecting the substance contained in the chamber.

Advantageously, the membrane is of the bi-stable type so as to independently maintain its own operating position between the operating positions described above.

In particular, such a membrane does not require any tools such as, for example, a plunger to maintain its position while using the container.

In addition, depending on the thickness of the membrane, both the intensities of a compressive force applied on the membrane and said bistability vary.

Moreover, the membrane has the form of a prolate or oblate spheroid or other form suitable for containing, advantageously, the collapse of the same into the shell.

Always in an advantageous way, the shell comprises a set of sharp-edged notches for perforating the membrane, in particular when in the second operating position, for ejecting the substance comprised in the container.

According to the invention, the shell also comprises grooves, facing in the direction of the chamber, to facilitate the ejection of the substance from the same chamber in which is contained.

Advantageously, the dispensing organ of the containment portion of the shell is connected to dispensing accessories such as, for example, needles.

Moreover, the shell comprises a support section for the fingers of a user.

In an advantageous way the container also comprises a movable activator device, having a shape corresponding to the shape of said shell, having a head adapted to push and compress said membrane inside the shell, in order to pass from the first operating position to the second position, and a body adapted to transmit the compression on the membrane during the use of the container.

Advantageously, the head of the activating device comprises a plurality of concentric slots for relieving it and for regulating the force necessary for deformation of the membrane.

Moreover, the head comprises cavities for facilitating the perforation of the membrane, by means of said sharp-edged notches present on the inner surface of the shell, preventing, in an advantageous way, the reuse of the container.

The head of the activator device also comprises sharp-edged notches capable of perforating the membrane after the relative compression, preventing reuse of the container.

In an advantageous way, the activator device comprises a toothed profile along the body wherein the pitch between the teeth of said toothed profile corresponds to a unit volume of the substance to be ejected.

**In** addition, the membrane and the activator device comprise, respectively, a coupling and a hole such that the coupling locks within the hole, to enable the membrane to be brought to the first operating position and to aspirate the substance into the chamber of the shell.

The membrane comprises also a protrusion, facing in the opposite direction of said coupling, the protrusion being apt to expel any substance remaining in the dispensing portion of the container.

Advantageously, the container comprises a ring for locking the membrane to said shell, wherein the ring is coupled to the shell and is apt to accommodate within it the activator device guiding its movement.

The shell comprises also a first portion tubular and hollow contiguous to the containment portion, wherein said ring is coupled, so as to fit within the first portion of the shell.

The membrane, comprises also an edge including a transverse portion terminating in a first apex, contiguous with and transverse to said transverse portion.

In further embodiments, the membrane comprises a second apex, contiguous and transverse to the transverse portion, wherein the second apex is adjacent and facing in the same direction as the first apex.

Moreover, the membrane comprises a third apex, transverse to the transverse portion, and positioned between the first and second apexes and facing the opposite direction from the same, to improve the tightness of the membrane.

The activator device comprises, advantageously, one or more rings for preventing the reuse of the container after use, preventing the return of the container to the first operating position.

In an advantageous way, the container also comprises a projecting element, suitable for switching from a rest position corresponding to the first operating position to an activation position corresponding to the second operating position in which, the projecting element is engaged with at least one of the one or more rings of the activating device, so as to secure it to the shell, to prevent its reuse.

The ring comprises, in preferred but not limiting embodiments, a projecting segment of a shape substantially complementary to said transverse portion of the membrane, and facing inwardly of the chamber, suitable for locking said transverse portion of the membrane at the shaping of the shell by holding it in place and acting as a seal.

Moreover, the ring comprises in a position contiguous with and opposite to the projecting segment a complementary flat segment and such as to contact the apex of the membrane.

Advantageously, the projecting segment and the flat segment of the ring are apt to respectively press the apex and the transverse portion of the membrane within the shaping of the shell.

In addition, the ring comprises, in a position opposite to the projecting segment a support preferably for the fingers of a user.

Furthermore, the present invention encompasses a production method of such a container as described above, as defined by the subject-matter of claims 25 and 26.

Advantageously, when in the production method the step of making the parts of the container, takes place by means of the injection and/or compression molding technique, during the production process, there is a step of controlling the cooling process of the parts so that the shaping and the transverse portion have a higher temperature than the rest of the membrane and the rest of the shell, so that the membrane sticks to the shell without the use of additional instrumentation.

Always in advantageous way, when in the production method the step of making the parts of the container, is carried out by means of the "Blow Fill Seal" (BFS) technique, during the manufacturing process, a portion of the shell is molded with a greater thickness than the remaining portion of the shell so as to make the shell rigid and a portion of the membrane with a lower thickness than the remaining portion of the membrane so as to make said membrane more flexible.

Moreover, when the step of making the parts of the container, takes place by means of the "Blow Fill Seal" (BFS) technique, during the production process, and before closing a mold, a shell-shaped insert is added to the container, wherein it comprises a containment portion and a dispensing organ.

In addition, when the step of realizing the parts of the container, takes place by means of the "Blow Fill Seal" (BFS) technique, during the production process, an insert in the form of a membrane suitable for being incorporated and/or welded on the shaping of the shell is added to said container, when still malleable.

Moreover, during the phase of realization of the parts of the container, when the moulding technique is used, there are the following further phases
As the arrangement of a plurality of shells within a molding die using a suitable nest for the arrangement of the shells in which, a heat-sealable film is positioned against the top of the shells being made,

Heating the edge of the shell and the film by a methodology and/or a combination of several methodologies such as, for example, induction, hot air blowing, use of infrared lamps, by convection;

Pressure of a mold against the molding die of the still malleable shells such that the film is welded to the molding of the shell;

Application of pressure difference at the shell and/or pressure at the delivery organ, on the molding die, such that, advantageously, the film deforms to form the relatively flexible membrane.

In addition, during the step of assembling the parts of the container the ring is positioned in such a way as to exert pressure between the transverse portion of the membrane and the shaping of the shell.

Advantageously, during the step of assembling the parts of the container, a layer of glue is arranged between the shaping of the shell and the portion of the membrane, to maintain the seal.

Moreover, during the step of assembling the parts of the container, in order to achieve a tight seal between the shaping and the transverse portion, the same are heated and pressed together by the application of an external pressure.

Advantageously, always during the step of assembling the parts of the container, ultrasound or infrared or other techniques known to promote mechanical engagement and interference between the parts are used to weld the portion of the membrane with the shaping of the shell.

In addition, prior the use of the container, it is filled with the substance and the toothed profile of the activator device is adapted to compress the membrane, eliminating the air present in the chamber of the container, so that the activator device locks in the first operating position.

### Brief description of the figures

Further features and advantages of the container for ejecting a substance according to the present invention will be more apparent from the following description, referring to a preferred and illustrative, but not limiting, embodiment and the accompanying drawings, wherein:
Figure 1A is a frontal sectional view showing the container for ejecting a substance in a first operating position wherein the container is ready for use, then before use thereof;
figure 1B is a frontal sectional view showing the container of figure 1A, in a second operating position, when in use;
figure 1C is a frontal sectional view showing the container of figure 1A, in a third end-use position;
Figure 1D is a detail view of the container of figure 1A in a first position of beginning use;
figure 1E is a view of a detail of the container of figure 1C in an end-use position;
Figures 2A to 2D show frontal and sectional views of further embodiments of the container membrane
Figure 2E shows a further embodiment of the membrane and container;
Figure 2F shows a detail relating to an improved system for sealing the membrane in the container;
Figure 3A shows a detail of the container, according to the present invention, with evidence of any disorderly states;
Figure 3B shows a detail relative to an embodiment of the shell belonging to the container, according to the present invention;
Figure 4A shows a perspective view of a further embodiment of the container;
figures 4B to 4E show details of an embodiment relating to the container of figure 4A;
Figures 5A and 5B show a frontal sectional view of a further embodiment of the container in two different positions of use;
Figures 6A and 6B show a frontal sectional view of a further embodiment of the container in the positions of initial use and end of use respectively;
Figure 6C shows a detail of the section depicted in Figure 6B;
Figures 7A-7C show frontal and sectional views of a further embodiment of the container;
Figures 8A and 8B show frontal and cross-sectional views of a further embodiment of the container in the initial and mid-use positions, respectively;
Figures 8C show a detail of the sectional view depicted in view 8A;
Figures 9A and 9B show one of the methods for producing the container;
Figures 10A to 10C show graphs depicting different trends in the deformation force of the container;
Figures 11A and 11B show a front sectional view of a further embodiment of the container 100;
Figure 12A shows a perspective view of a further embodiment of the container when the BFS process is used;
Figure 12B shows a front sectional view of the container of figure 12A;
Figure 12C shows a graph of the thickness control of the container parts, used by the BFS process of figure 12A;
Figures 13A -13C show frontal sectional views of three further embodiments of the container using the BFS process with inserts.

### Detailed description

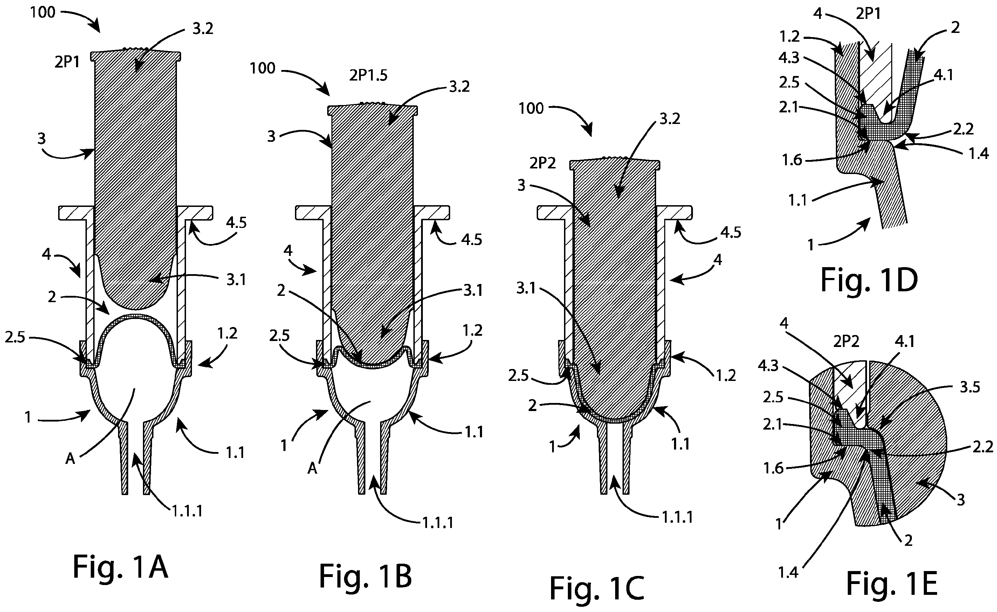

With reference to Figures 1A to 1C, a first embodiment of the container for ejecting a substance, generically referred to by the numerical reference 100, is observed.

The container 100, in preferred but not limiting embodiments, is a syringe suitable for the containment and ejection of at least one substance, in particular medicaments.

Advantageously, in contrast to the prior art described above, the container 100 object of the present invention does not have any piston for dispensing the substance and thus becomes more reliable.

Advantageously, the container 100 has a very simple design comprising a hollow shell 1 for the containment and ejection of a substance and a deformable membrane 2, wherein said shell 1 is substantially more rigid than said membrane 2 mentioned above.

Further, the container 100 comprises, in preferred but not limiting embodiments, a plunger 3 capable of deforming the membrane 2 for ejection of the substance and a ring 4 for locking said membrane 2 to the shell 1, wherein the ring 4 is also capable of guiding the movement of the plunger 3 within the container 100.

The shell 1, as visible in the figures, comprises a first portion 1.2 of preferably tubular and hollow shape and a second containment portion 1.1 of concave , which is contiguous with and has a smaller diameter than the first portion 1.2.

As can be seen in the figures, the second portion 1.1 of the shell 1 has a substantially "cup" shape, e.g. hemispherical, ending, at the central/base point of the "cup", with a dispensing organ 1.1.1. Still advantageously, in further embodiments of the container 100, which is the object of the present invention, said dispensing organ 1.1.1. can be arranged in an off-centre position with respect to the central point/base of the "cup", in particular at the side of the shell 1.

Advantageously, the dispensing organ 1.1.1 is contiguous to the second containment portion 1.1 and is hollow and of oblong shape, to allow dispensing of the substance contained in the container 100 itself.

Advantageously, the containment portion 1.1 and, in particular, the associated dispensing organ 1.1.1, in preferred, but not limiting, embodiments, is connected to accessories, such as a needle, for dispensing the substances contained within the container 100. Furthermore, the dispensing organ 1.1.1, prior to use of the container 100, is capped by a cap (not visible in the illustrated embodiments), which cap is removed prior to use allowing, if necessary, any accessories for dispensing the substance such as a needle or connector to be attached.

Further, between the first portion 1.2 and the second portion 1.1 the container 100 advantageously comprises a shaping 1.6 connecting the two portions, so that the second portion 1.1 is projecting internally, by a section 1.4 of the shell 1, with respect to the first portion 1.2. Advantageously, said section 1.4 goes from a first edge facing inwards of the container 1, to a second edge facing outwards.

In particular, the shaping 1.6 results in a surface that is contiguous and transverse with respect to the first portion 1.2, wherein the second edge of the shaping 1.6 has substantially the same inner diameter as the first portion 1.2.

Advantageously, a membrane 2 is placed at the surface of the aforementioned shaping 1.6.

Advantageously, since the shell 1 can be made by means of different manufacturing techniques such as, by way of example only, blow moulding, moulding, injection moulding, compression moulding, BFS, thermoforming or 3D printing, it is made of plastic or substantially elastic and/or plastic materials.

Again with reference to the embodiment under consideration, the membrane 2 is substantially concave and made so as to be deformable, turnable and/or bi-stable. By bi-stable, it is meant that the membrane 2 maintains position independently and therefore does not require the plunger 3 to be held in position or to be squeezed to match the shell 1, as will be explained below.

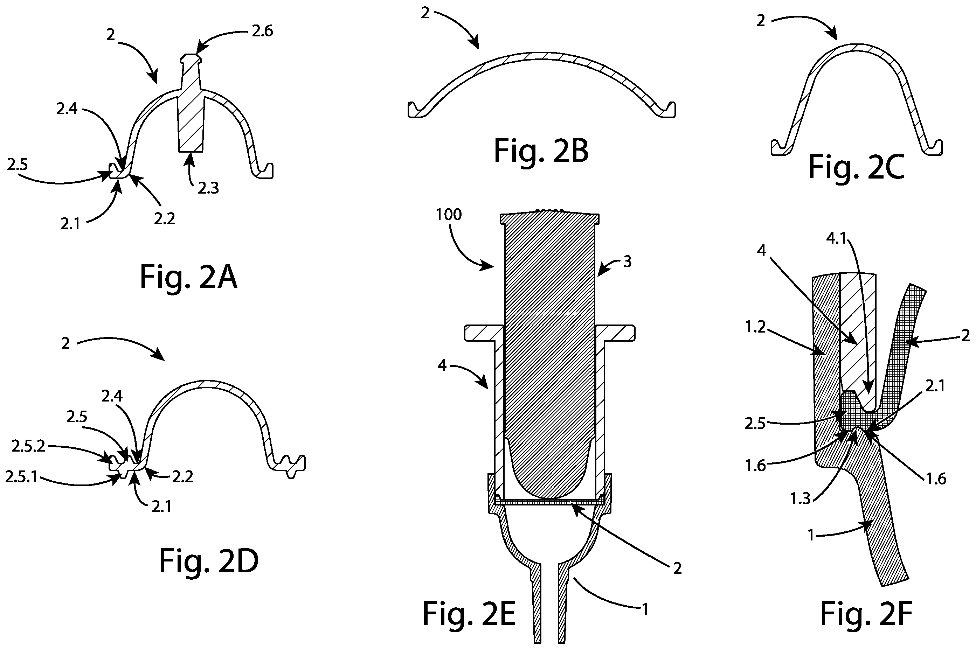

In detail, in the embodiment under consideration, the membrane 2 has a substantially prolate spheroid shape and is connected to the shell 1, as visible in Figures 1A, 1B and 1C, in correspondence with the relative shaping 1.6. Advantageously, said membrane 2 may have, as required, other shapes or configurations such as, by way of example only, those depicted in Figures 2B and 2D, wherein the same has a shape substantially of a prolate spheroid or an oblate spheroid.

Advantageously, similarly to the shell 1, the membrane 2 is made of elastic and/or plastic materials and is shaped in such a way that the shape of the shell 1 coincides with that of the membrane 2 to allow, as will be seen later, a complete dispensing of what is contained in the container 100.

Advantageously, the membrane 2 may be circular, rectangular, square or any shape, whether regular or irregular. In detail, the shape of the membrane 2 is such that it advantageously influences the operation of the container 100 in its entirety, e.g. in the case where the membrane 2 is designed in the shape of a prolate spheroid, as visible in figure 2C, the force required for its compression in the direction of the shell 1 and, therefore, the force to be applied on it by the plunger 3 will be greater than in the case where the membrane 2 is designed in the shape of an oblate spheroid (figure 2B).

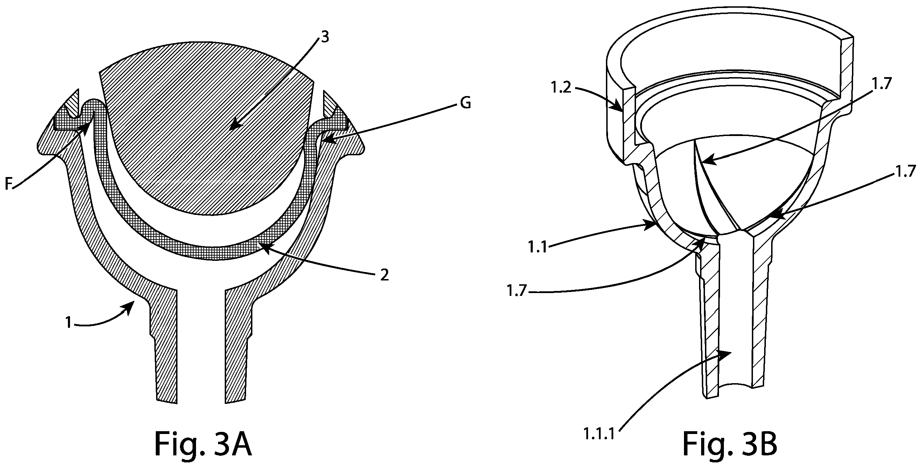

In particular, factors such as the thickness, elasticity, hardness and tensile strength of the membrane 2 are physical properties that determine the possible formation of folds F on the surface of the membrane 2 itself and/or possible gaps G (Figure 3A) formed during the deformation process of the membrane 2 towards the shell 1 by the plunger 3.

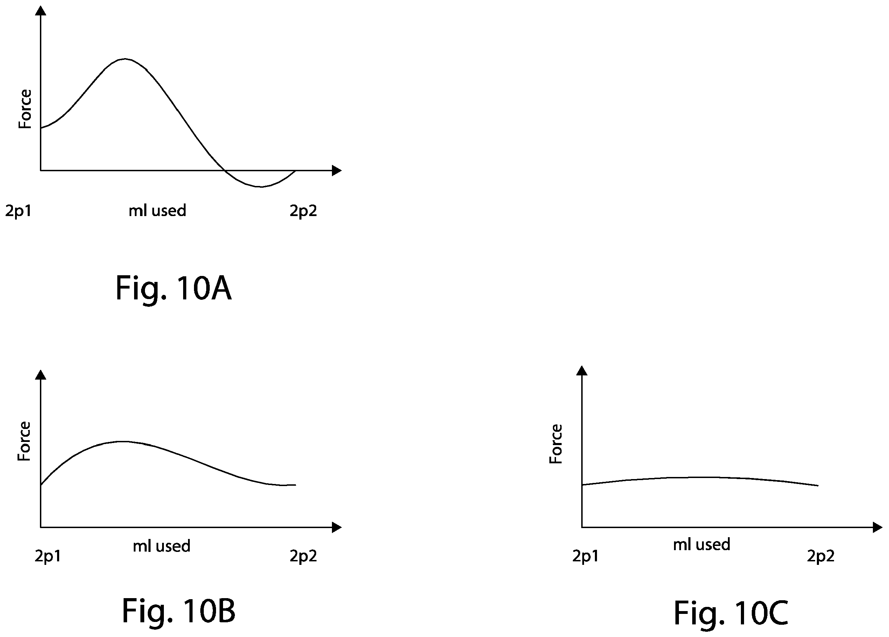

If Membrane 2 is made so that it is thicker at its central area, so that the thickness gradually decreases as it moves away from the centre, the force required to compress it will be almost constant (fig. 10C); conversely, if Membrane 2 is uniformly thick and rigid, the force profile to move Plunger 3 will not be as constant (fig. 10A-10B).

Further, as visible in Figures 1D and 1E, said membrane 2 comprises a transverse portion 2.1 in correspondence with the relative edge of the concavity, which transverse portion 2.1 terminates with an apex 2.5, contiguous and transverse to the transverse portion 2.1 itself, for the attachment and connection of the membrane 2 to the shell 1 in correspondence with the relative shaping 1.6, so that the membrane 2 can be configured in the different operating positions. Furthermore, contiguous to each transverse portion 2.1, on the opposite side with respect to the apex 2.5, the membrane 2 comprises an angled portion 2.2 suitable, due to its structure, to change angle and concavity during the different phases of use and, therefore, consistent with the use of the container 100, as visible in Figure 1E.

Advantageously, as seen in Figure 2D, in further embodiments but not limitation, the membrane 2 comprises a second apex 2.5.2, contiguous and transverse to the portion 2.1 itself, in particular, positioned adjacent and facing the same side as the apex 2.5.

Furthermore, again advantageously, the membrane 2 further comprises a third apex 2.5.1, transverse to the portion 2.1, positioned between the first and second apexes 2.5 and 2.5.2, but facing in the opposite direction of the aforementioned first and second apexes 2.5 and 2.5.2.

In particular, the above-mentioned design of membrane 2 is intended to improve the tightness of membrane 2 itself.

In detail, it is through the ring 4 and the shaping 1.6 that the membrane 2 is calibrated. In particular, since the ring 4 and the shaping 1.6 are substantially rigid with respect to the membrane 2, they press it at the transverse portion 2.1, calibrating it and advantageously creating a seal between the parts.

Advantageously, as mentioned above, the membrane 2 is bi-stable, in fact it has at least two operating positions with respect to the shell 1: an initial position 2p1 (Figure 1A) and an end-use configuration 2p2 (Figure 1C); in addition to these two positions, advantageously, the membrane 2 could be in any other intermediate position between them (Figure 1B).

In the first configuration 2p1, the membrane 2 is made with a shape such that it coincides with the shape of the shell 1 when turned upside down. Said membrane 2 is placed with opposite concavity with respect to the shell 1, structurally realising a hollow chamber A suitable for the containment of liquids, gases or any other substance to be dispensed. It should be noted that several substances or mixtures of substances, such as, by way of example, air and/or other gases, to be dispensed at the same time, can also be placed in said hollow chamber A.

In particular, when the container 100 is in the second end position configuration 2p2 (Fig. 1C), since the membrane 2 is shaped in such a way as to adhere to the shell 1, when the membrane 2 is compressed by the plunger 3, the same collapses until it substantially matches the shell 1, in the direction of the compression so as to eject all the contents present in the chamber A. Again advantageously, the membrane 2 is made to coincide perfectly, when collapsed, with the geometry of the inner surface of the shell 1, so as to eject all the liquid present in the chamber A.

Advantageously, as mentioned above, the membrane 2 may also be of the bi-stable type and thus not need to be held in place by the plunger 3 and to be squeezed so as to fit the shell 1

Further, in order to eject the substance contained in chamber A, the plunger 3 must exert a force on the membrane 2 that varies depending on the thickness, elasticity, hardness, tensile strength and shape of the membrane 2 and the back pressure realised by the dispensing portion 1.1.

More specifically, advantageously, using a membrane 2 with a thickness profile adjusted so that the force required to move the plunger 3 is within the prescribed range also allows the thickness profile of the membrane 2 to be designed so that the minimum and maximum force to move the plunger 3 is within the prescribed range.

Advantageously, in the end position 2p2 (Fig. 1E), when the membrane has been deformed by the plunger 3, totally on the shell 1 of the container 100, the angled portion 2.2 of the membrane 2 will assume a certain radius which, if it is coincident with that of the section 1.4 of the shell 1 will not structurally allow the creation of a space G between the two and therefore nothing can be trapped between them (Figures 1D and 1E).

In addition, the membrane 2 is designed in such a way that it is deformed, only as an example, by the plunger 3, to move at least between the two configurations 2p1 and 2p2.

In figure, 1B, membrane 2 is shown as a partially deformed membrane. Particularly, depending on the diameter of the membrane 2, the depth of the movement of the plunger 3 to squeeze the contents out of the chamber A and the back pressure generated in the chamber A during the forward movement of the plunger 3, the membrane 2 tends to fold. For the reasons mentioned above, it is essential to adjust the geometry of the plunger rod 3 to allow the necessary space to accommodate the folding of diaphragm 2. In fact, if such an adjustment of the geometry of the plunger 3 is not made, the force to move the plunger 3 could rapidly increase to the point of making it impossible to use the syringe.

Generally, a key element is that in any disposable syringe it is important to expel all contents at the end of the dispensing process.

In a syringe with a membrane, it is intuitive to match the size and shape of the plunger with the shape of the shell. On the other hand, this attempt to match the shape of the plunger with the shape of the shell to squeeze the liquid may be at odds with the need to provide a space to accommodate the folds of the membrane when the plunger advances to expel the liquid.

More specifically, the stability of the contents of the container, for example the pharmaceutical formulation contained in chamber A, depends on gas exchange and the tendency of the contents to migrate from chamber A to the external environment. This leads to a disadvantage because, polymeric and elastomeric materials are permeable to gases and the substance or formulation. Therefore, if shell 1 and membrane 2 are not thick enough, the product contained in chamber A may not achieve the desired stability.

Furthermore, stability is also a function of the surface area/volume ratio of chamber A. Of all geometric shapes, the spherical shape is the most efficient as the smallest surface area can contain the maximum volume. Any deviation from the spherical shape of chamber A leads to a reduction in the stability of the contents compared to the spherical shape, provided that the other variables and the thickness of the membrane and shell remain unchanged.

For the above reasons, nowadays, in order to reduce production costs, reduce the carbon footprint and reduce leachates (additives and volatiles contained in the polymer that are released in the formulation) there is a tendency to reduce thickness, so the spherical shape may help, but the elastomeric membrane, being more permeable, needs a certain thickness to maintain the stability of the formulation contained in the A chamber. The minimum thickness for elastomeric membranes used on the market today should be 0.2 mm.

In addition, nowadays most syringes, whether pre-filled or single-use empty, are equipped with a standard luer port on which a needle with a hub can be mounted. In more detail, the standard luer port has a small hole to allow the passage of the formulation contained in chamber A when the plunger is pushed to eject the substance contained in the container.

A known issue of the prior art is that when the membrane is lying in its initial position or initial configuration 2P1, it is not easy to fill chamber A of the syringe through such a small hole because the air that is displaced has to escape through the same hole.

To facilitate filling, the membrane 2, according to the present invention, can be collapsed by applying vacuum to the dispensing organ or port or by applying compressed air to the other side of the membrane 2. In this collapsed form of membrane 2, the volume of chamber A is reduced to zero and thus chamber A can be filled without the need to vent air, because there is no air in chamber A when membrane 2 is collapsed. This has the advantage of greatly reducing filling times, and filling chamber A when it is empty also reduces the chances of the substance escaping.

Advantageously, as can be seen in Figures 1A-1E, the container 100 also has a ring 4 located in correspondence with the first portion 1.2 of the shell 1, and such that it extends in a direction opposite to the dispensing organ 1.1.1 of the shell 1.

In particular, the ring 4 has dimensions such as to fit at least partially within the first portion 1.2 of the shell 1.

The portion 1.2 of the shell 1 and the corresponding area of the ring 4 are coupled using various methods having the purpose of joining them; such methods may be, for example, welding, gluing, mechanical coupling, interference, etc.

The ring 4, as visible in Figures 1D and 1E advantageously comprises a first projecting segment 4.1 of a shape substantially complementary to the transverse portion 2.1 of the membrane 2 and facing inwards from the chamber A of the shell 1, substantially from the side that will contact the plunger 3.

Furthermore, contiguous to and on the opposite side with respect to the first projecting segment 4.1, the ring 4 further comprises a flat segment 4.3 complementary to and such as to contact the apex 2.5 of the membrane 2.

Additionally, the ring 4, in a position opposite the end facing the second portion 1.1 of the shell 1, comprises a support 4.5 for the finger, which is in contact with the plunger 3 to facilitate the use of the container 100 itself. Advantageously, the ring 4, is capable of guiding the sliding of the plunger 3 inside the container 100, possibly also blocking its rotation.

More in detail (Fig. 1D), the ring 4 is apt to accommodate within it the plunger 3, in particular during its movement in the direction of the shell 1, and to press and block the transverse portion 2.1 of the membrane 2 at the shaping 1.6 of the shell 1, holding it in position. Further, in other preferred embodiments, in order to create sealing between the shell 1 and the membrane 2, the membrane can not only be compressed by the ring 4, as described above, but advantageously, it can be sealed by further welding/joining.

As can be seen in Figure 1D, the first projecting segment 4.1 and the flat segment 4.3 of the ring 4 are shaped in such a way that they simultaneously and respectively press the apex 2.5 and the transverse portion 2.1 of the membrane 2 inside the shaping 1.6 of the shell 1, holding the membrane 2 in place and acting as a seal thereof. Advantageously, the dimensions of ring 4, membrane 2 and shell 1 are such that the pressure exerted by ring 4 must not exceed the elastic modulus of membrane 2.

Advantageously, the plunger 3, as visible in Figures 1A, 1B and 1C has a substantially ogival shape and comprises a head 3.1 apt to push and compress the membrane 2 inside the shell 1 for the ejection of the substance contained in the container 100, during its use, and a body 3.2 of oblong shape apt to be placed in correspondence with the ring 4 and apt to transmit the force on the membrane during the use of the container 100.

Said plunger 3 may be made, for example, of plastic, and/or by moulding and/or other fabrication techniques.

In particular, the shape of the head 3.1 of the plunger 3 is substantially complementary to that of the shell 1, so that, during operation of the container 100, the same can push the membrane 2 causing it to completely overlap, since it has the same geometry as the interior of the shell 1, causing the substance contained therein to completely spill out.

The geometry of the head 3.1 of the plunger can be realised to be able to adjust the force required to deform the membrane.

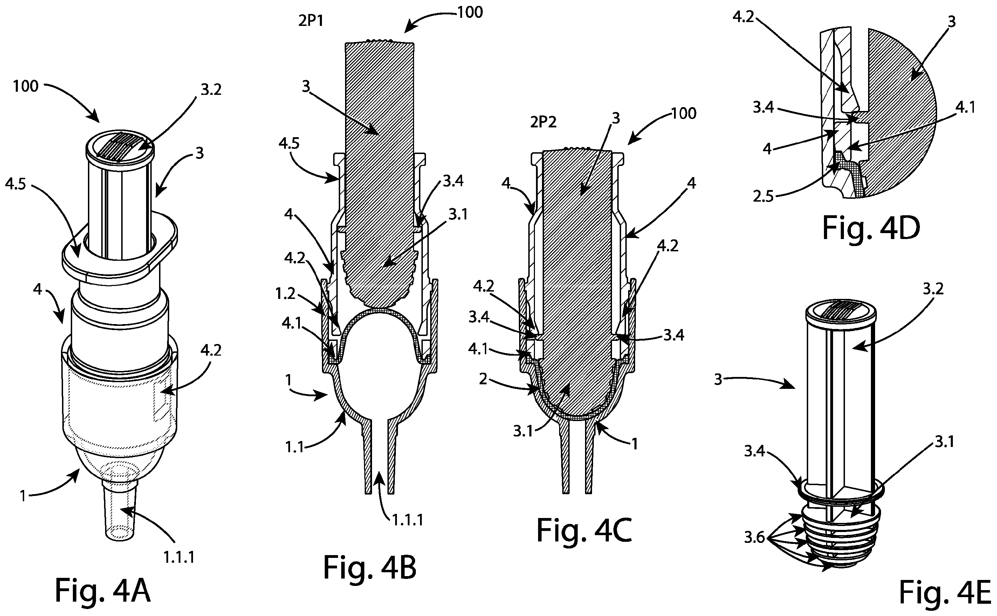

Advantageously, the head 3.1 of the plunger 3, in preferred embodiments as seen in Figures 4E may have a plurality of concentric slots 3.6 to reduce the weight of the plunger 3 and to, advantageously, lighten the weight of the plunger itself and adjust the force required for deformation of the membrane. In addition, plunger 3 is triggerable via a finger/thumb of the user.

The shell 1, in a further embodiment, as visible in Figure 2F, substantially in correspondence with the shaping 1.6, as described above, includes a protruding portion 1.3 which, due to its geometry, is apt to improve the seal obtained with the transverse portion 2.1 of the membrane 2. In particular, advantageously, the geometry of the protruding portion 1.3 is projecting and facing in the direction of the transverse portion 2.1 of the membrane 2. Preferably, said projecting portion 1.3 has a curved profile.

Further, the shell 1, as visible in figure 3B, may comprise grooves 1.7 facing in the direction of the chamber A, which are intended to allow the substance contained in the container to flow out, to prevent it from being trapped in the points G of disorder, as described above and as visible in figure 3A.

In a further embodiment, as visible in Figures 4A to 4D, the ring 4 comprises a second protruding lever segment 4.2 which, after use of the container 100, contacts the plunger 3, in such a way as to secure it to the shell 1, making it impossible to remove it. In detail, the plunger 3 has, along the body 3.2, one or more rings 3.4 to prevent reuse of the container 100 after use by preventing the container from returning to the starting operating position 2p1 and, therefore, acting as a coupling to the second protuding segment 4.2 lever of the ring 4.

Advantageously, such second protruding lever segment 4.2 structurally results on the inside from the first portion 1.2 of the shell 1, so as to be inaccessible from the outside. Advantageously, this makes tampering with the protruding element 4.2 impossible.

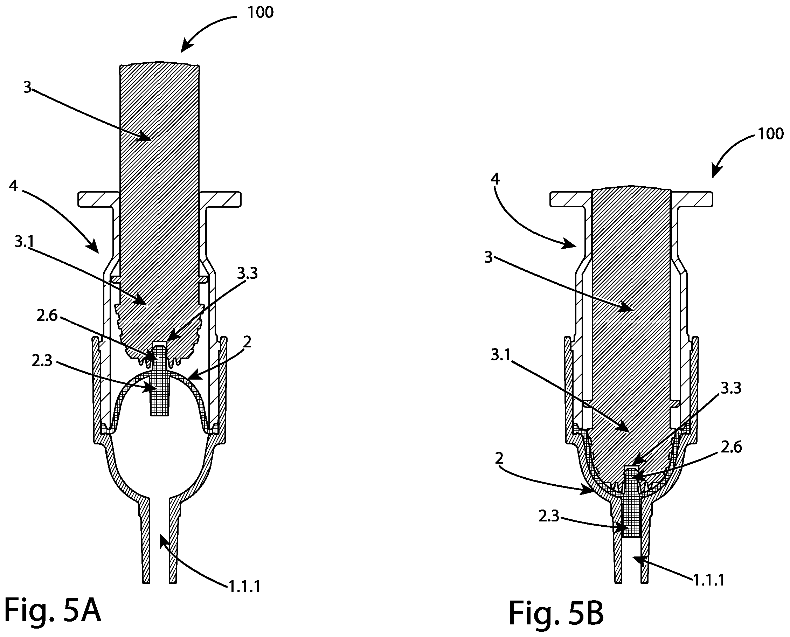

In further preferred embodiments of the container 100, as visible in Figures 5A and 5B, the membrane 2 and the plunger 3 comprise, respectively, a coupling 2.6 and a hole 3.3; in detail, the geometry of the coupling 2.6 and the hole 3.3 is such as to allow the coupling 2.6 to be locked inside the hole 3.3, not allowing it to be unlocked. This embodiment may be used to allow the membrane 2 to move according to the first operating position, i.e. in the opposite direction to the concavity of the shell 1, creating a depression in the chamber A. In particular, this form of embodiment allows the substance to be sucked into the chamber A of the shell 1, in the event that the substance is to be sucked from the container 100, for example, by means of a movement in the opposite direction to that of the dispensing given by the plunger 3.

Additionally, the membrane 2 may have a protrusion, in particular a cap 2.3, facing in the opposite direction of the coupling 2.6, such that it can be used to squeeze the liquid trapped in the dispensing organ 1.1.1, ensuring that all of the substance is ejected from the container 100.

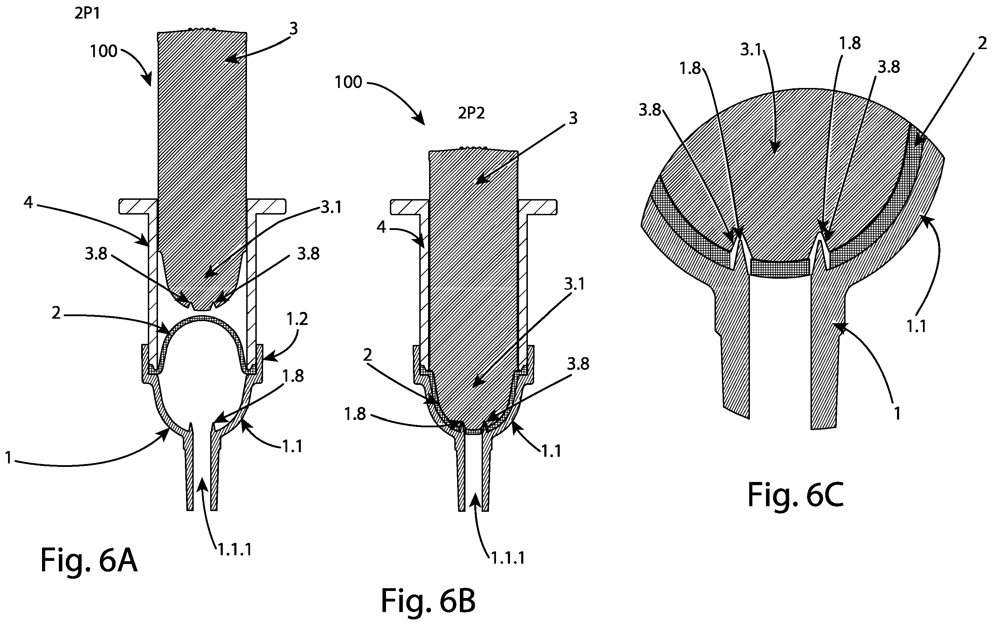

In further detail, in a further embodiment, the plunger 3, as visible in Figures 6A to 6C, in correspondence with the respective head 3.1 also comprises cavities 3.8, while the shell 1, in correspondence with the said cavities 3.8 has, in its respective inner surface, sharp-edged notches 1.8, for cutting the membrane 2, following the use of the container 100, in particular, in its end-use The presence of the aforementioned notches 1.8 advantageously allows the container 100 to be prevented from being reused as they are capable of permanently puncturing the membrane 2.

Alternatively, in further embodiments, the series of sharp-edged notches can be positioned on the head 3.1 of the plunger 3; in this case, the shell 1 has holes to allow the membrane 2 to stretch and break on the aforementioned notches of the head 3.1, to prevent reuse of the device.

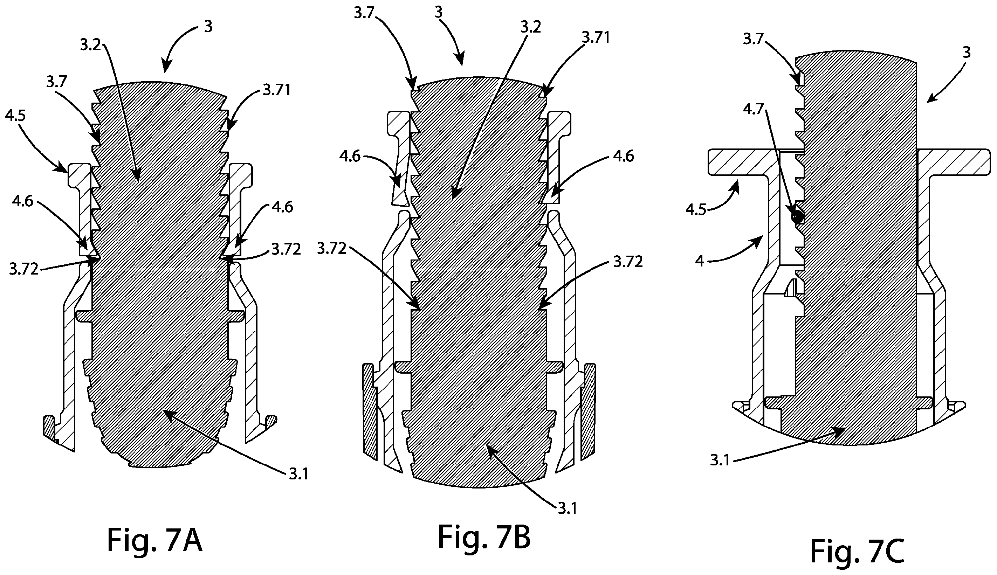

In a further embodiment of the container 100, as visible in Figures 7A and 7B, the ring 4 may comprise a plurality of hooks 4.6 projecting and facing into the container 100 and suitable for contacting a toothed profile comprising a plurality of teeth 3.7 and 3.71 which are substantially arranged on the body 3.2 of the plunger 3.

In detail, at the body 3.2, the plunger 3 comprises a toothed profile, wherein the teeth 3.7, 3.71 are configured to act as a control relative to the dose of substance to be ejected from the container 100. In detail, advantageously, the distance between the teeth corresponds to a unit of volume of substance to be ejected and is such as to maintain said volume constant during ejection: the more teeth there are, the more clicks there are and, for this reason, this type of configuration could be used, advantageously, to have tactile and/or auditory feedback on the flow/dose of substance ejected. Additionally, again advantageously, the aforementioned toothed profile, in preferred designs, is suitable for removing air present in chamber A, for example after filling the container. In particular, as the toothed profile exerts pressure on the membrane 2 it is such as to compress it, removing the air present in the chamber A and locking it in position, as explained above, to advantageously allow the container 100 to be used correctly.

If the teeth 3.7, 3.71 of the toothed profile are of the sharp-edged type, as visible in Figures 7A-7C, they prevent the movement of the plunger 3 in the opposite direction to the direction of ejection, such movement possibly caused, for example, by pressure in chamber A.

The teeth 3.7, 3.71, in another embodiment, can be of the rounded type, also allowing the movement of the plunger 3 in the opposite direction with respect to the direction of ejection, only creating a feedback system that allows the user a better precision in dosing the substance contained in the container 100.

In order to increase the overall number of clicks the teeth 3.7 and 3.71 can be offset between one side and the other of the body 3.2 of the plunger 3, as visible in Figures 7A and 7B. To avoid stress on the hooks 4.6, the body 3.2 has aligned teeth 3.72 when in position 2P1.

Another embodiment of the ring 4, shown in Figure 7C, the ring 4 has a transverse through-hole which allows the passage of a wire 4.7, realising a mechanism similar to that discussed in the previous embodiment.

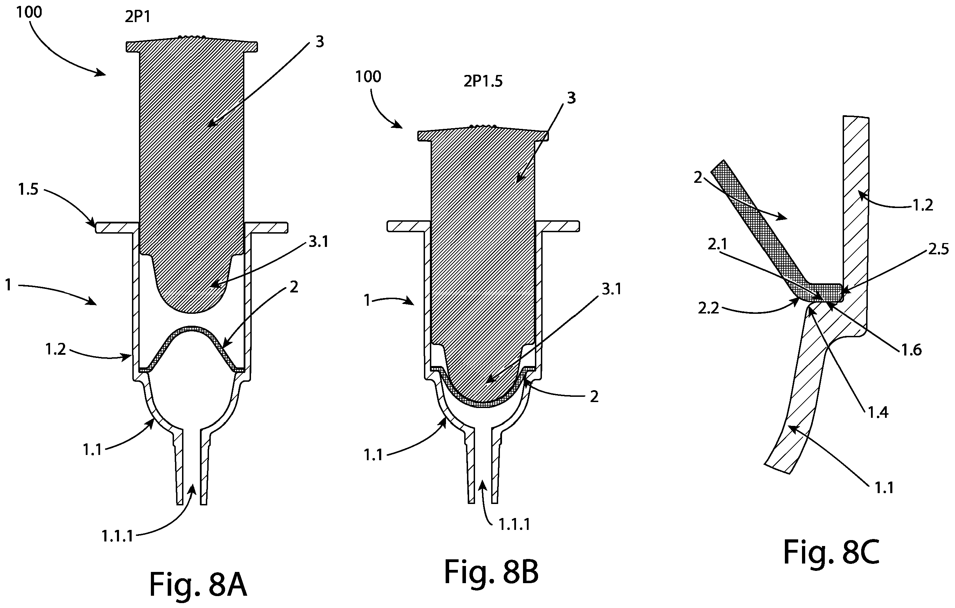

A further embodiment, shown in Figures 8A to 8C, the shell 1 has the first portion 1.2 more elongated than in the previous embodiments, so as to present a support section 1.5, suitable to act as a support for the fingers of a user.

By way of example only, said support section 1.5 is projecting in the direction of the outside of the container 100.

Advantageously, in the embodiment under analysis, the ring 4 is not present and the membrane 2 is coupled to the shell 1 between the shaping 1.6 and the transverse portion 2.1, for example, by a heat welding method.

In this embodiment, the membrane 2 after arriving at the intermediate position 2P1.5 is stretched by the movement of the plunger 3 itself; this allows the membrane 2 to adhere better to the inner surface of the shell 1 causing less liquid to remain inside after use.

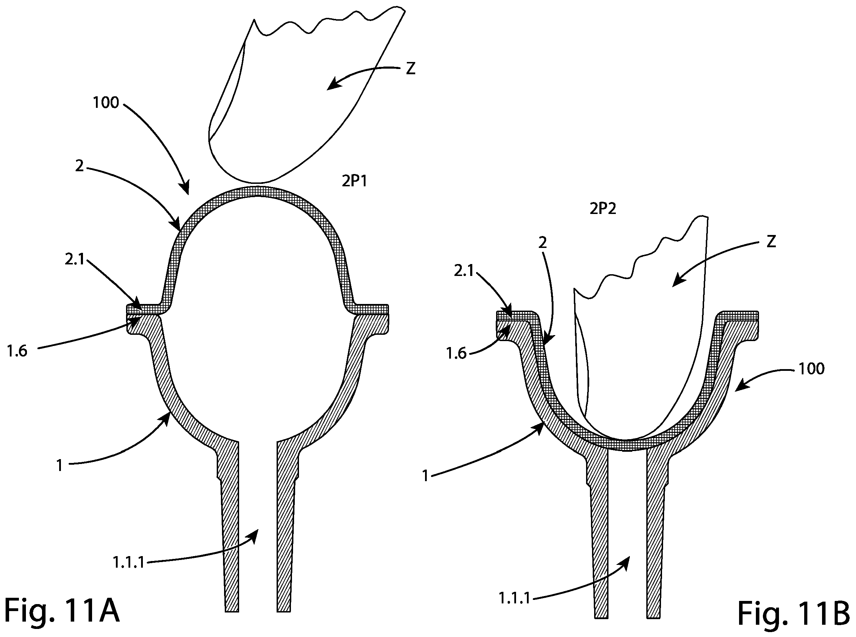

A further embodiment of the container 100, as visible in Figures 11A and 11B, is characterized by the absence of the plunger 3. The membrane 2 and the shell 1 are coupled using the methods described above such as, for example, heat welding or gluing. Further, again in the embodiment form under analysis of the container 100, it may have the ring 4 so as to form the seal between the membrane 2 and the shell 1. The advantage of this embodiment is that the membrane is made with a bi-stable shape, which allows the membrane to remain in place even when the user removes the finger Z after use.

Further, in an embodiment of the container 100, not shown in the figures, the membrane 2 is deformable by means of a spring.

In particular, this embodiment is advantageous for the following reasons.

Shell 1, plunger rod 3, ring 4 and dispensing organ 1.1.1 all occupy a certain volume. Plunger rod 3 and ring 4 should be at least twice as long as the stroke required to move the entire volume contained in chamber A. Thus, by providing for a plunger rod 3 to eject the contents from chamber A, it is necessary for the container 100 to provide at least 3 times the volume of chamber A.

For these reasons, advantageously, the work of ejecting the contents can also be carried out with the aid of a compressed conical spring which, once released, can expand to eject the contents from chamber A, without contributing significantly to the volume occupied by the container 100. Such a spring-activated container 100 will occupy a volume slightly greater than that of the spherical chamber A, resulting in a significant reduction in volume compared to that required by the container 100 with the plunger rod 3.

Advantageously, the spring could be shaped and designed in such a way that, when extended, it assumes the contour of the membrane 2 in its stable position, so that the contour of the extended spring helps to eject the entire contents of chamber A. This leads to a reduction in secondary volume and a reduction in secondary and tertiary packaging material and transport costs. This volume saving is highly appreciated in the case of vaccines, which are transported in the cold chain and the space occupied by the syringe is an issue.

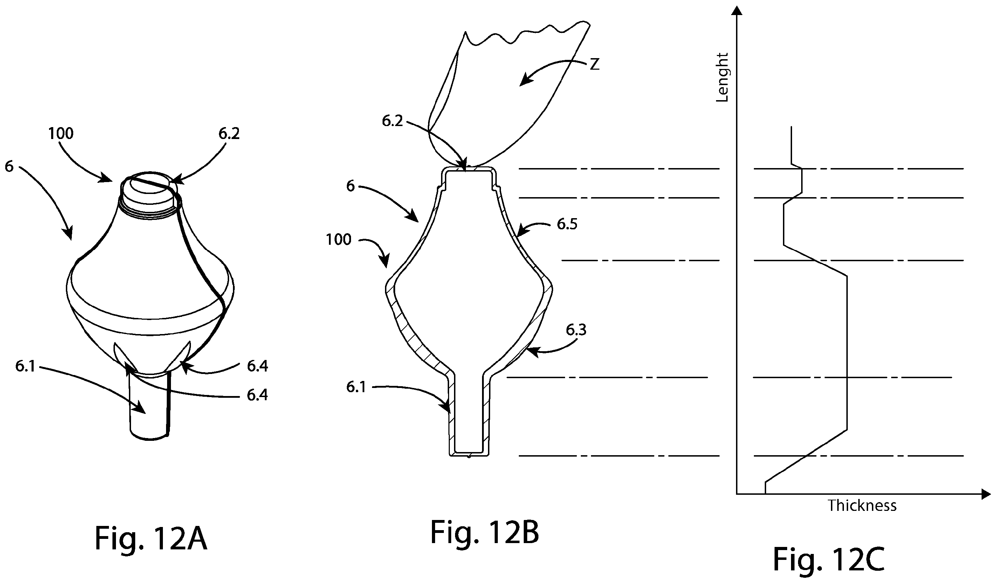

A further embodiment of the container 100 is shown schematically in Figures 12A and 12B. In detail, the container 100, unlike the previous embodiments, does not include any membrane and is made in one piece 6 using, for example, the BFS (Blow - Fill - Seal) system.

In particular, the container 100 has a first zone 6.1 suitable for allowing the substance to exit, which may have a threaded connection or a luer insert or various shapes suitable for dispensing/ejecting the substance contained within the container 100.

Additionally, in a position opposite the first zone 6.1 there is a second zone 6.2 from which, advantageously, the filling of the container 100 can take place during the respective creation process and from which the compression of the container through, for example, the finger Z takes place.

As is particularly visible in Figures 12B and 12C, the advantage of this embodiment is inherent in the fact that, structurally, the container 100 comprises two different thicknesses. In particular, a first area 6.3 having a first thickness, which is placed substantially above the first zone 6.1 and, a second area 6.5 having a second thickness, which is less than the first thickness, which is placed directly below the second zone 6.2 of the container 100.

In particular, this difference in thickness between the parts of the container 100, is also accentuated by possible reinforcements 6.4 placed radially on the first area 6.3 and around the first zone 6.1 of the container. In particular, this difference in thickness means that, the zone of lower thickness, which is placed in correspondence with the second area 6.5, can deform in the direction of the interior of the container 100, functioning in a similar way to the mechanism of operation of the membrane 2, as described in the previous embodiments, in which the membrane 2 is apt to collapse inside the shell 1. Variation in the thickness of the container is enabled by controlling the thickness of the parison, which follows a profile as described in Figure 12C.

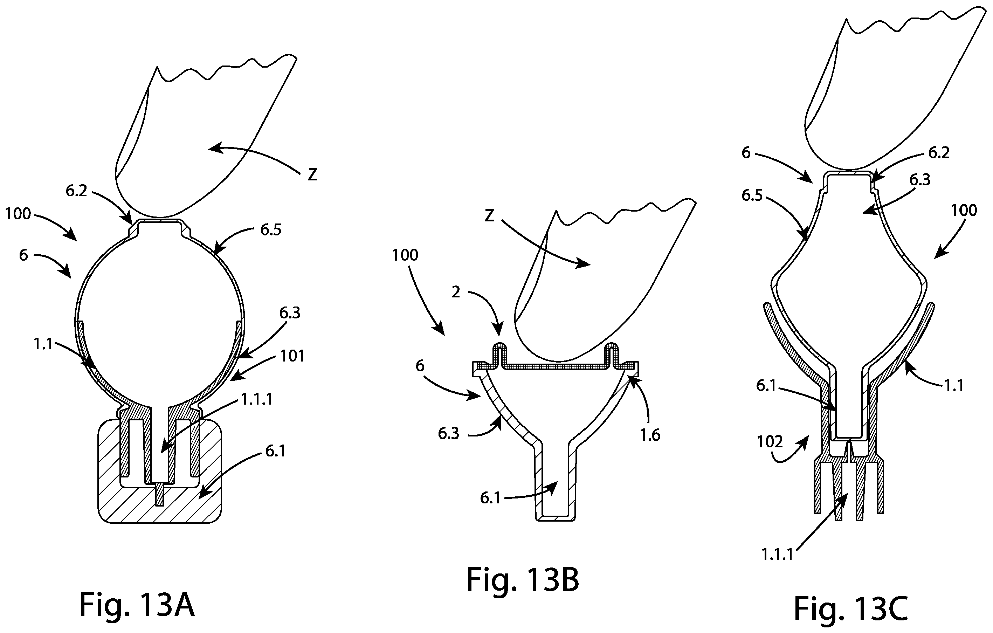

Two further embodiments are illustrated in Figures 13A and 13B, in which the container is fabricated using the BFS process by adding an insert during the manufacturing process.

In Figure 13A, the shell-shaped insert 101 is inserted during the BFS process and, in particular, before the mould is closed. The shell 101, similarly to what has been described in previous embodiments is composed of a containment portion 1.1, for example hemispherical, and a dispensing organ 1.1.1 to which various dispensing systems can be applied.

The first zone 6.1 of the container 100 made of BFS serves as the cap of the container 100, which must be removed before use to allow ejection of the substance contained therein. Following the insertion phase of the shell 101 as described, the filling phase of the container 100 takes place through the opening located in the second zone 6.2, which is then sealed by the subsequent closing of the mould heads to form a closed container.

The advantage of this form of embodiment over the previous form of embodiment, described in Figures 12A and 12B, is that a drastic change in thickness between the areas 6.3 and 6.5 is not necessary since the shell 1 acts as a reinforcement of the area 6.3. Advantageously, area 6.5 is apt to collapse when crushed, similar to that described above.

Figure 13B shows the other embodiment of the container 100, in which the insert that is inserted during the BFS process is the membrane 2. The container 100 is made in open BFS, and when the latter is still malleable the membrane 2 is incorporated and/or welded onto the moulding 1.6.

Finally, a further embodiment, depicted in Figure 13C, shows the container 100 comprising an outer rigid shell 102 and a closed container 6 made from BFS. In correspondence with the dispensing organ 1.1.1 of the shell 102 there is a coupling system designed to connect the dispensing organ 1.1.1 with the first zone 6.1 of the container 6. In particular, the container 100 in order to be used according to the aforementioned embodiment, must be activated, i.e. the container 6 is pushed towards the shell 102 and the portion 1.1 comes into contact with the area 6.5 of the container acting as a reinforcement.

The container 100, which is the object of the present invention, functions substantially as follows. As mentioned above, in the first configuration 2P1 in which the container 100 is closed (container 100 full, prior to ejection of the substance) the membrane 2, having substantially the same geometry as the shell 1, is placed with opposite concavity with respect to the same, structurally generating the hollow chamber A containing the substance to be dispensed. Subsequently, the plunger 3, by means of an external pressure acting in the direction of the shell 1 itself, begins to gradually compress the membrane 2. At this point, through the compression of the plunger 3, the membrane 2 gradually changes concavity while simultaneously pushing the substance contained within the chamber A towards the dispensing organ 1.1.1. Chamber A begins to empty until the membrane 2 collapses, adhering substantially to the inner surface of the shell 1.

In particular, when the container 100 is in the second end-use configuration 2P2, as the membrane 2 is shaped to coincide with the shell 1, the membrane 2 is compressed by the plunger 3, and collapses back to substantially match the shell 1, in the direction of compression so as to eject all the contents present in the chamber A.

Advantageously, in the end position 2P2, when the membrane has been compressed by the plunger 3 totally on the shell 1 of the container 100, the angled portion 2.2 of the membrane 2 will assume a certain radius which, if it is coincident with that of the section 1.4 of the shell 1, will not structurally permit the creation of a space G between the two and therefore nothing can be trapped in this position.

Today, there are two main methods for producing elastomeric membranes: injection moulding and vulcanisation. Although the hemispherical membrane remains stable in two positions, the position in which it has the least internal tension is the position in which it is unmoulded by the injection mould or vulcanisation mould.

This state of minimum internal stress can be further improved by annealing the membrane after moulding.

In this state of minimum stress, the elastic membrane will not form creases but will faithfully maintain the contours that the mould imprints on the membrane during the moulding process. We call this the first stable position of the bistable membrane.

When this membrane is turned into its second stable position, stresses are generated in the membrane, because the inner part of the membrane tends to compress and the outer part of the membrane tends to stretch. As a result of these stresses, dents, dimples and ridges appear on the surface of the membrane.

Thus, according to the present invention, in the initial position or first use configuration 2P1 of the container 100, when the chamber A is fully stretched, if the membrane 2 is already turned to its second stable position and the plunger 3 is pushed to eject the contents of the chamber A, the membrane 2 returns to its first stable position in which it was unstretched. Since this is the position where residual stresses in membrane 2 are minimal, it tends to assume its original profile. Thus, without dents, dimples and ridges, the membrane will expel all the liquid contained in chamber A. **In** fact, in the absence of surface irregularities, membrane 2 tends to adhere to the surface of shell 1 even when the shape of plunger 3 does not correspond exactly to that of shell 1.

Advantageously, the following table gives limit values, as an example, to ensure that the maximum amount of substance content in chamber A can be squeezed through the dispensing apparatus 1.1.1, for a container filling volume of 0.2 ml to 100 ml.

The thickness of the membrane 2 and the surface area of the membrane 2 should be such that the force required for squeezing remains within the desired limits.

The following table gives the maximum and minimum values for membrane thickness and surface area.

| PARAMETER | LIMIT VALUES (Maximum and minimum) | COMMENT |
|---|---|---|
| One or more of the dimensions of a square or rectangular membrane (not the thickness ) | From 5 mm to 70 mm | If we assume that the membrane is square or a rectangle, the dimension of the membrane of the smallest container with a filling volume of 0.05 ml could be 5 mm x 5 mm |
| If the shape of the membrane is round, its diameter must be between: | From 5 mm to 70 mm | |
| Surface area of the membrane in contact with content of chamber A | From 10 mm2 ti 6000 mm2 | If we assume that the membrane is circular, the diameter could be between 5 mm and 70 mm. The smallest container with a membrane diameter of 5 mm could have a filling volume of 0.1 ml. |
| Membrane perimeter if the membrane is not round, rectangular or square | From 10 mm to 150 mm | Considering that the 100 ml syringe might have a 70 mm diameter membrane. |
| Membrane thickness | From 0,1 mm to 5 mm | The thickness may be different if the syringe is designed to aspirate the liquid |
| Membrane hardness in Shore A or D | From 10 to 150 | For container filling volume between 0.05 ml and 100 ml |
| Diametro dello stantuffo se ha circonferenza rotonda | From 3 mm to 70 mm | For syringe filling volume between 0.05 ml and 100 ml |
| Plunger size if not round. | 3 mm to 100 mm | |
| Tensile strength of membrane material | 1Mpa to 85Mpa | For syringe filling volume between 0.05 ml and 100 ml |
| Membrane material | Any Polymer, Elastomer, TPE, TPV complying with current regulations and compatible with the intended content. | |
| Materials that can be used with the BFS process | Polypropylene, polyethylene, EVA copolymer. | Shore hardness depends on filling volume A higher shore hardness is required if the syringe is designed to aspirate liquid. |
| If the circumference of the membrane is round, the ratio of its height to the radius (h / r) is | 0.2 - 5 | |

Generally, the production method for the realization of the container 100, which is the object of the present invention, consists of one or more of the following steps:
- Making the parts of the container, in detail: shell 1, membrane 2, plunger 3 and ring 4, by one or more of the following processes: blow moulding, injection moulding, compression moulding, BFS, thermoforming or 3d printing.
- Assembly of the above parts in a suitable/sterile environment;
- Secondary packaging of the container in a suitable environment
- Terminal sterilization of the container using one or more of the known sterilization methods.

The container 100, which is the object of the present invention, is realisable by means of different methods; the fundamental element of the production process is that it is realised in such a way that a good seal is advantageously created between the membrane 2 and the shell 1. Advantageously, this optimal seal between the two elements is created by the pressure exerted by the ring 4.

Alternatively, to maintain the seal between the shaping 1.6 of the shell 1 and the membrane 2, a layer of glue can be advantageously provided.

Yet another method to achieve a tight seal between the two elements is to heat the shaping 1.6 and the transverse portion 2.1 and then apply external pressure to it (Fig. 8C).

Another method would be to make the 2.5 portion of the membrane 2 so that it interferes with the 1.2 portion of the shell 1.

With regard to injection and/or compression moulding, an innovative step would be to advantageously control the tooling cooling process in such a way that the weld zone, such as for example the shaping 1.6, and the cross-sectional portion, such as for example the transverse portion 2.1, can remain relatively warm compared to the rest of the membrane 2 and the rest of the shell 1.

In detail, after such controlled cooling, when the shell 1 and the membrane 2 have been formed, the transverse portion 2.1 of the membrane 2 and the shaping 1.6 of the shell 1 still have a relatively higher temperature than the other parts of the container 100, by way of example only a temperature above 60°C, so advantageously, the membrane 2 when coupled to the shell 1 welds without the use of additional mechanical instrumentation or without the need to heat the parts prior to coupling.

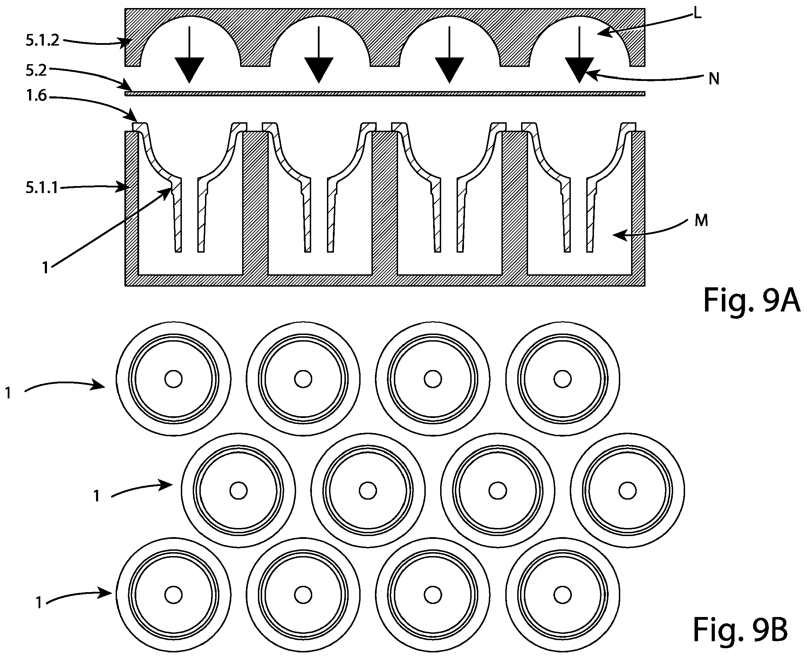

A further innovative production method could be to arrange several shells 1 in a matrix, as shown in Figure 9A, using a suitable nest 5.1.1. In detail, a heat-sealable film 5.2 is then placed against the top of the shells 1 being manufactured, wherein only the shaping 1.6 of the shell 1 and the film 5.2 are heated.

Advantageously, one or a combination of several known methods may be used to heat said heat-sealable film 5.2, such as, but not limited to, induction, hot air blowing, the use of infrared (IR) lamps, by convection or by any other means/methodology suitable for heating.

After heating, the mould can be pressed against the still malleable moulding die of the shells 1 to weld the film 5.2 with the shaping 1.6 of the shell 1.

A pressure difference is applicable both at the level of the nest 5.1.1 and at the cavity L of the mould 5.1.2 which is placed above the nest 5.1.1. In this way, the film 5.2 will deform to form the relatively flexible membrane 2.

With particular reference to Figures 12A, 12B and 12C, another method that can be used to make the container 100 is the "Blow Fill Seal" (BFS), a manufacturing process that consists of extruding a tubular parison of suitable polymer to mould the rigid shell 1 and membrane 2, filling the container 100 with the substance and sealing the container from the top of the mould in the zone 6.2. Advantageously, as mentioned above, the innovative step of the "Blow Fill Seal" process consists in moulding the part of the shell with a greater thickness to make the shell 1 relatively rigid and the part of the membrane 2 with a lower thickness to make it more flexible using, for example, automatic parison thickness control.

The invention thus conceived and illustrated herein is susceptible to numerous modifications and variations, all within the scope of the inventive concept.

Furthermore, all details may be replaced by other technically equivalent elements.

Finally, the components used, as long as they are compatible with the specific use, as well as the dimensions, may be varied according to requirements and the state of the art.

Where features and techniques mentioned in any claim are followed by reference marks, those reference marks have been included for the sole purpose of increasing the intelligibility of the claims and, consequently, those reference marks have no limiting effect on the interpretation of each element identified by way of example by those reference marks.

## Claims

1. Container (100) for ejecting a substance, such as medicaments and the like, comprising
A hollow shell (1) for the containment and ejection of said substance, including
a containment portion (1.1) comprising a dispensing organ (1.1.1) of oblong shape,
a membrane (2), connected to said shell (1),
said container (100) comprising, in a position contiguous to and transverse with respect to said containment portion (1.1), a shaping (1.6), for connecting said membrane (2), so that said membrane (2) can move from
a first operating position, in which the membrane (2) has a central portion in a position opposite to the containment portion (1.1) of the shell (1), creating with the containment portion (1.1) a chamber (A) for the containment of said substance, to
a second operating position, wherein the central portion of the membrane (2) collapses within said portion (1.1) of containment of said shell (1), so as to eject said substance present in the chamber (A) through said dispensing organ (1.1.1)
wherein
the membrane (2) is subject to folds (F) and/or gaps (G) formed during the deformation process,
**characterized in that** said shell (1) comprises grooves (1.7), facing in the direction of said chamber (A), to facilitate the ejection of said substance from said chamber (A),
the grooves (1.7) allowing the substance contained in the container to flow out, and to prevent the substance from being trapped in the folds (F) and/or gaps (G).

2. Container (100) as in claim 1, **characterized in that** said membrane (2) is calibrated with the geometry of said containment portion (1.1) of said shell (1), so as to adhere to and be compressible in the direction of said shell (1), for ejecting said substance contained in said chamber (A).

3. Container (100) according to claim 1 or 2, **characterized in that** said membrane (2) is of the bi-stable type so as to independently maintain its own operating position between said operating positions.

4. Container (100) according to one of claims 1-3, **characterized in that** depending on the thickness of said membrane (2) the intensities of a compressive force applied on said membrane (2) and said bistability vary.

5. Container (100) according to one of claims 1-4, **characterized in that** said membrane (2) has the form of a prolate or oblate spheroid or other form suitable for containing said substance and collapsing into the shell (1).

6. Container (100) according to claim 5, **characterized in that** said shell (1) comprises a set of sharp-edged notches (1.8) for perforating said membrane (2) in the second operating position, for ejecting said substance.

7. Container (100) according to one of claims 1-6, **characterized in that** said dispensing organ (1.1.1) of said containment portion (1.1) of said shell (1) is connected to dispensing accessories such as, for example, needles.

8. Container (100) according to one of the claim 1-7, **characterized in that** said shell (1) comprises a support section (1.5) for the fingers of a user.

9. Container (100) according to one of the claims 1-8, **characterized in that** it comprises a movable activator device (3), having a shape corresponding to the shape of said shell (1), having a head (3.1) adapted to push and compress said membrane (2) inside said shell (1), in order to pass from said first operating position to said second position, and a body (3.2) adapted to transmit said compression on said membrane (2) during the use of the container (100).

10. Container (100) as in claim 9, **characterized in that** said head (3.1) of said activating device (3) comprises a plurality of concentric slots (3.6) for relieving said device (3) and for regulating the force necessary for deformation of said membrane (2).

11. Container (100) according to claims 6 and 9, **characterized in that** said head (3.1) comprises cavities (3.8) for facilitating the perforation of said membrane (2), by means of said sharp-edged notches (1.8) present on the inner surface of said shell (1), preventing the reuse of said container (100).

12. Container (100) as to at least one of claims 9-11, **characterized in that** said head (3.1) of the activator device (3) comprises sharp-edged notches capable of perforating said membrane (2) after relative compression, preventing reuse of said container (100).

13. Container (100) as to at least one of claims 9-12, **characterized in that** said activator device (3) comprises a toothed profile (3.7, 3.71) along the body (3.1) wherein the pitch between the teeth of said toothed profile (3.7, 3.71) corresponds to a unit volume of said substance to be ejected.

14. Container (100) according to one of the claims 1-13, **characterized in that** the membrane (2) and the activator device (3) comprise, respectively, a coupling (2.6) and a hole (3.3) such that said coupling (2.6) locks within said hole (3.3) to enable the membrane (2) to be brought to the first operating position and to aspirate said substance into the chamber (A).

15. Container (100) according to claim 14, **characterized in that** the membrane (2) comprises a protrusion (2.3), facing in the opposite direction of the coupling (2.6), wherein said protrusion (2.3) being apt to expel the substance remaining in the dispensing portion (1.1.1).

16. Container (100) according to claim 9, **characterized in that** it comprises a ring (4) for locking said membrane (2) to said shell (1) wherein said ring is coupled to said shell (1) and is apt to accommodate within it the activator device (3) guiding its movement, wherein said shell (1) comprises a first portion (1.2) tubular and hollow contiguous to said containment portion (1.1), wherein said ring (4) is coupled, so as to fit within the first portion (1.2) of said shell (1).

17. Container (100) according to one of the claims 1-16, **characterized in that** said membrane (2), comprises an edge including a transverse portion (2.1) terminating in a first apex (2.5), contiguous with and transverse to said transverse portion (2.1).

18. Container (100) according to claim 17, **characterized in that** said membrane (2) comprises a second apex (2.5.2), contiguous and transverse to said portion (2.1), wherein said second apex (2.5.2) is adjacent and facing in the same direction as said apex (2. 5), and a third apex (2.5.1), transverse to the portion (2.1), positioned between the first and second apexes (2.5, 2.5.2), being facing the opposite direction from said first and second apexes (2.5, 2.5.2), to improve the tightness of said membrane (2).

19. Container (100) according to claim 9, **characterized in that** said activator device (3) comprises, along the body (3.2), one or more rings (3.4) for preventing the reuse of the container (100) after use, preventing the return of the container (100) to the first operating position.

20. Container (100) according to claim 19, **characterized in that** it comprises a projecting element (4.2), suitable for switching from a rest position corresponding to said first operating position to an activation position corresponding to said second operating position in which, the projecting element (4.2) is engaged with at least one of said one or more rings (3.4) of said activating device (3), so as to secure it to the shell (1), to prevent its reuse.

21. Container (100) according to claim 16 and 17, **characterized in that** said ring (4) comprises a projecting segment (4.1) of a shape substantially complementary to said transverse portion (2.1) of the membrane (2), and facing inwardly of said chamber (A), suitable for locking said transverse portion (2.1) of the membrane (2) at the shaping (1.6) of the shell (1) by holding it in place and acting as a seal.

22. Container (100) according to claim 21, **characterized in that** said ring (4) comprises in a position contiguous with and opposite to said projecting segment (4.1) a complementary flat segment (4.3) and such as to contact said apex (2.5) of said membrane (2).

23. Container (100) according claim 22, **characterized in that** the projecting segment (4.1) and said flat segment (4.3) of the ring (4) are apt to respectively press the apex (2.5) and the transverse portion (2.1) of the membrane (2) within the shaping (1.6) of the shell (1).

24. Container (100) according to claim 21, **characterized in that** said ring (4) comprises, in a position opposite to the projecting segment (4.1) a support (4.5) preferably for the fingers of a user.

25. Production method of a container (100) according to any one of the preceding claims, comprising the following steps:
A. Making the parts of said container (100), by injection molding and/or compression molding wherein said parts comprise the shell (1) and the membrane (2);
B. Assembly of said parts in a suitable, sterile environment; Secondary packaging of the container (100) in a suitable environment;
C. Terminal sterilization of the container (100) using one or more of the known methods of sterilization
**characterized in that** during the production process there is a step of controlling the cooling process of said parts so that the shaping (1. 6) and the transverse portion (2.1) have a higher temperature than the rest of the membrane (2) and the rest of the shell (1), so that the membrane (2) sticks to the shell (1) without the use of additional instrumentation.

26. Production method of a container (100) according to any one of the preceding claims, comprising the following steps:
A. Making the parts of said container (100), by BFS, by extruding a tubular parison of suitable polymer to mould the shell (1) and membrane (2);
B. Assembly of said parts in a suitable, sterile environment;
Secondary packaging of the container (100) in a suitable environment;
**characterized in that**, during the manufacturing process, a portion of the shell (1) is molded with a greater thickness than the remaining portion of the shell (1) so as to make the shell (1) rigid and a portion of the membrane (2) with a lower thickness than the remaining portion of the membrane (2) so as to make said membrane (2) more flexible.

27. Production method according to claim 26 **characterized in that**, during the production process, and before closing a mold, a shell-shaped insert (101) is added to said container, wherein said insert (101) comprises a containment portion (1.1) and a dispensing organ (1.1.1).

28. Production method according to claim 25 **characterized in that**, during the phase of realization of said parts of said container (100), when the moulding technique is used, there are the following further phases
Arrangement of a plurality of shells (1) within a molding die using a suitable nest (5.1.1) for the arrangement of said shells (1) in which, a heat-sealable film (5.2) is positioned against the top of the shells (1) being made,
Heating the edge of the shell (1) and the film (5.2) by a methodology and/or a combination of several methodologies such as, for example, induction, hot air blowing, use of infrared lamps, by convection;
Pressure of a mold (5.1.2) against the molding die of the still malleable shells (1) such that the film (5.2) is welded to the shaping (1.6) of the shell (1);
Application of pressure difference at the shell (1) and/or pressure at the delivery organ (1.1.1), on the molding die, such that the film (5.2) deforms to form the relatively flexible membrane (2).

29. Production method according to claim 25 or 28, **characterized in that**, during the step of assembling the parts of said container (100) the ring (4) is positioned in such a way as to exert pressure between the transverse portion (2.1) of the membrane and the shaping (1.6) of the shell (1).

30. Production method according to claims 25 or 28-29 **characterized in that**, during the step of assembling said parts of the container (100), a layer of glue is arranged between the shaping (1.6) of the shell (1) and the portion (2.1) of the membrane (2), to maintain the seal.

31. Production method according to one of claims 25 or 28-29, **characterized in that** during the step of assembling said parts of the container (100), in order to achieve a tight seal between the shaping (1.6) and the transverse portion (2.1), said shaping (1.6) and said transverse portion (2.1) are heated and pressed together by the application of an external pressure.

32. Production method according to at least one of claims 25 or 28-29, **characterized in that**, during the step of assembling said parts of said container (100), ultrasound or infrared or other techniques known to promote mechanical engagement and interference between said parts are used to weld the portion (2.1) of the membrane (2) with the shaping (1.6) of the shell (1).

33. Production method as to at least one of the claim 25 or 26 of a container (100) according to claim 13, **characterized in that**, prior to use, said container (100) is filled with said substance and said toothed profile of said activator device (3) is adapted to compress said membrane (2), eliminating the air present in said chamber (A) of said container (100), so that said activator device (3) locks in said first operating position.

## Patentansprüche

1. Behälter (100) zum Ausstossen einer Substanz, wie beispielsweise Arzneimittel und dergleichen, mit
Eine Hülse (1) zur Aufnahme und zum Ausstoßen der genannten Substanz, umfassend
ein Aufnahmeraum (1.1), der ein Ausgabeglied (1.1.1) von länglicher Form umfasst,
eine mit dem Gehäuse (1) verbundene Membran (2), wobei der Behälter (100) an einer Stelle, die an den Aufnahmeraum (1.1) angrenzt und quer zu diesem verläuft, eine Formung (1.6) zum Verbinden der Membran (2) aufweist, so dass sich die Membran (2) von eine erste Betriebsstellung, in der sich der zentrale Abschnitt der Membran (2) gegenüber dem Aufnahmesegment (1.1) der Hülle (1) befindet und mit dem Aufnahmesegment (1.1) eine Kammer (A) zur Aufnahme der Substanz bildet, um
eine zweite Betriebsstellung, in der der mittlere Abschnitt der Membran (2) in den Aufnahmeraum (1.1) des Gehäuses (1) eintaucht, um die in der Kammer (A) befindliche Substanz durch die Abgabevorrichtung (1.1.1) auszustoßen wobei
die Membran (2) weist Falten (F) und/oder Spalten (G) auf, die während des Verformungsprozesses entstanden sind,
**dadurch gekennzeichnet, dass** die Hülle (1) in Richtung der Kammer (A) weisende Rillen (1.7) aufweist, um das Austreten der Substanz aus der Kammer (A) zu erleichtern,
die Nuten (1.7), die es ermöglichen,
damit die im Behälter enthaltene Substanz herausfließen kann und verhindert wird, dass sich die Substanz in den Falten (F) und/oder Spalten (G) festsetzt.

2. Behälter (100) nach Anspruch 1, **dadurch gekennzeichnet, dass** die Membran (2) an die Geometrie des Aufnahmesektors (1.1) der Hülle (1) angepasst ist, sodass sie an dieser anliegt und in Richtung der Hülle (1) komprimierbar ist, um die in der Kammer (A) enthaltene Substanz auszustoßen.

3. Behälter (100) nach Anspruch 1 oder 2, **dadurch gekennzeichnet, dass** die Membran (2) vom bistabilen Typ ist, um ihre eigene Betriebsposition unabhängig zwischen den genannten Betriebspositionen beizubehalten.

4. Behälter (100) gemäß einem der Ansprüche 1-3, **dadurch gekennzeichnet, dass** sich die Stärke der auf die Membran (2) ausgeübten Druckkraft und die Bistabilität in Abhängigkeit von der Dicke der Membran (2) ändern.

5. Behälter (100) gemäß einem der Ansprüche 1-4, **dadurch gekennzeichnet, dass** die Membran (2) die Form eines länglichen oder abgeflachten Sphäroids oder eine andere Form aufweist, die geeignet ist, die Substanz aufzunehmen und sich in die Hülle (1) zusammenzuziehen.

6. Behälter (100) nach Anspruch 5, **dadurch gekennzeichnet, dass** die Hülle (1) eine Reihe scharfkantiger Kerben (1.8) aufweist, um die Membran (2) in der zweiten Betriebsposition zu durchstechen und so die Substanz auszustoßen.

7. Behälter (100) gemäß einem der Ansprüche 1-6, **dadurch gekennzeichnet, dass** das Abgabeorgan (1.1.1) des Aufnahmeteils (1.1) der Hülle (1) mit Abgabevorrichtungen, wie beispielsweise Nadeln, verbunden ist.

8. Behälter (100) nach einem der Ansprüche 1-7, **dadurch gekennzeichnet, dass** die Hülle (1) einen Stützabschnitt (1.5) für die Finger eines Benutzers aufweist.

9. Behälter (100) nach einem der Ansprüche 1-8, **dadurch gekennzeichnet, dass** er eine bewegliche Betätigungsvorrichtung (3) umfasst, deren Form der Form des Gehäuses (1) entspricht und die einen Kopf (3.1) aufweist, der dazu ausgelegt ist, die Membran (2) im Inneren des Gehäuses (1) zu drücken und zusammenzudrücken, um von der ersten Betriebsposition in die zweite Position überzugehen, sowie einen Körper (3.2) aufweist, der dazu ausgelegt ist, die Kompression auf die Membran (2) während der Verwendung des Behälters (100) zu übertragen.

10. Behälter (100) nach Anspruch 9, **dadurch gekennzeichnet, dass** der Kopf (3.1) der Betätigungsvorrichtung (3) mehrere konzentrische Schlitze (3.6) aufweist, um die Vorrichtung (3) zu entlasten und die zur Verformung der Membran (2) erforderliche Kraft zu regulieren.

11. Behälter (100) gemäß den Ansprüchen 6 und 9, **dadurch gekennzeichnet, dass** der Kopf (3.1) Vertiefungen (3.8) aufweist, die das Durchstechen der Membran (2) mittels der an der Innenfläche der Hülle (1) vorhandenen scharfkantigen Kerben (1.8) erleichtern und so die Wiederverwendung des Behälters (100) verhindern.

12. Behälter (100) gemäß mindestens einem der Ansprüche 9-11, **dadurch gekennzeichnet, dass** der Kopf (3.1) der Aktivierungsvorrichtung (3) scharfkantige Kerben aufweist, die nach einer relativen Kompression die Membran (2) durchstechen können und so eine Wiederverwendung des Behälters (100) verhindern.

13. Behälter (100) nach mindestens einem der Ansprüche 9-12, **dadurch gekennzeichnet, dass** die Auslösevorrichtung (3) ein Zahnprofil (3.7, 3.71) entlang des Körpers (3.1) aufweist, wobei die Teilung zwischen den Zähnen des Zahnprofils (3.7, 3.71) einem Volumeneinheit der auszutreibenden Substanz entspricht.

14. Behälter (100) nach einem der Ansprüche 1-13, **dadurch gekennzeichnet, dass** die Membran (2) und die Aktivierungsvorrichtung (3) jeweils eine Kupplung (2.6) und eine Öffnung (3.3) aufweisen, so dass die Kupplung (2.6) in der Öffnung (3.3) einrastet, um die Membran (2) in die erste Betriebsposition zu bringen und die Substanz in die Kammer (A) anzusaugen.

15. Behälter (100) nach Anspruch 14, **dadurch gekennzeichnet, dass** die Membran (2) einen Vorsprung (2.3) aufweist, der in die der Kupplung (2.6) entgegengesetzte Richtung weist, wobei der Vorsprung (2.3) dazu geeignet ist, die im Ausgabebereich (1.1.1) verbleibende Substanz auszustoßen.

16. Behälter (100) nach Anspruch 9, **dadurch gekennzeichnet, dass** er einen Ring (4) zum Verriegeln der Membran (2) an der Hülle (1) umfasst, wobei der Ring mit der Hülle (1) verbunden ist und dazu ausgelegt ist, in seinem Inneren die Aktivierungsvorrichtung (3) aufzunehmen, die deren Bewegung führt, wobei die Hülle (1) einen ersten Abschnitt (1.2), der rohrförmig und hohl ist und an den Aufnahmeteil (1.1) angrenzt, wobei der Ring (4) so verbunden ist, dass er in den ersten Abschnitt (1.2) des Gehäuses (1) passt.

17. Behälter (100) nach einem der Ansprüche 1-16, **dadurch gekennzeichnet, dass** die Membran (2) einen Rand aufweist, der einen Querabschnitt (2.1) umfasst, der in einer ersten Spitze (2.5) endet, die an den Querabschnitt (2.1) angrenzt und quer zu diesem verläuft.

18. Behälter (100) nach Anspruch 17, **dadurch gekennzeichnet, dass** die Membran (2) einen zweiten Scheitelpunkt (2.5.2) aufweist, der an den Abschnitt (2.1) angrenzt und quer zu diesem verläuft, wobei der zweite Scheitelpunkt (2.5.2) an den Scheitelpunkt (2. 5) sowie einen dritten Scheitelpunkt (2.5.1), der quer zu dem Abschnitt (2.1) verläuft und zwischen dem ersten und dem zweiten Scheitelpunkt (2.5, 2.5.2) angeordnet ist, wobei er in die entgegengesetzte Richtung zu dem ersten und dem zweiten Scheitelpunkt (2.5, 2.5.2) weist, um die Dichtheit der Membran (2) zu verbessern.

19. Behälter (100) nach Anspruch 9, **dadurch gekennzeichnet, dass** die Auslösevorrichtung (3) entlang des Körpers (3.2) einen oder mehrere Ringe (3.4) aufweist, um die Wiederverwendung des Behälters (100) nach Gebrauch zu verhindern und zu verhindern, dass der Behälter (100) in die erste Betriebsposition zurückkehrt.

20. Behälter (100) nach Anspruch 19, **dadurch gekennzeichnet, dass** er ein vorstehendes Element (4.2) umfasst, das dazu geeignet ist, von einer Ruhestellung, die der ersten Betriebsstellung entspricht, in eine Aktivierungsstellung zu wechseln, die der zweiten Betriebsstellung entspricht, in der das vorstehende Element (4.2) mit mindestens einem der einen oder mehreren Ringe (3.4) der Aktivierungsvorrichtung (3) in Eingriff steht, um diese an der Hülle (1) zu sichern und so deren Wiederverwendung zu verhindern.

21. Behälter (100) gemäß den Ansprüchen 16 und 17, **dadurch gekennzeichnet, dass** der Ring (4) einen vorstehenden Abschnitt (4.1) aufweist, dessen Form im Wesentlichen komplementär zu dem Querabschnitt (2.1) der Membran (2) ist, und nach innen in Richtung der Kammer (A) weist, das dazu geeignet ist, den Querabschnitt (2.1) der Membran (2) an der Formung (1.6) der Schale (1) zu arretieren, indem es diesen festhält und als Dichtung wirkt.

22. Behälter (100) nach Anspruch 21, **dadurch gekennzeichnet, dass** der Ring (4) an einer Stelle, die an das vorstehende Segment (4.1) angrenzt und diesem gegenüberliegt, ein komplementäres flaches Segment (4.3) aufweist, das so ausgebildet ist, dass es die Spitze (2.5) der Membran (2) berührt.

23. Behälter (100) nach Anspruch 22, **dadurch gekennzeichnet, dass** das vorstehende Segment (4.1) und das flache Segment (4.3) des Rings (4) so ausgebildet sind, dass sie die Spitze (2.5) bzw. den Querabschnitt (2.1) der Membran (2) in die Formgebung (1.6) der Schale (1) drücken.

24. Behälter (100) nach Anspruch 21, **dadurch gekennzeichnet, dass** der Ring (4) an einer dem vorstehenden Abschnitt (4.1) gegenüberliegenden Stelle eine Stütze (4.5) aufweist, vorzugsweise für die Finger eines Benutzers.

25. Verfahren zur Herstellung eines Behälters (100) gemäß einem der vorstehenden Ansprüche, das die folgenden Schritte umfasst:
A. Herstellung der Teile des genannten Behälters (100) durch Spritzgießen und/oder Formpressen, wobei die genannten Teile die Hülle (1) und die Membran (2) umfassen;
B. Montage der genannten Teile in einer geeigneten, sterilen Umgebung;
Sekundärverpackung des Behälters (100) in einer geeigneten Umgebung;
C. Endsterilisation des Behälters (100) unter Verwendung eines oder mehrerer bekannter Sterilisationsverfahren
**dadurch gekennzeichnet, dass** der Herstellungsprozess einen Schritt umfasst, bei dem der Abkühlvorgang der genannten Teile so gesteuert wird, dass der Formteil (1.6) und der Querabschnitt (2.1) eine höhere Temperatur aufweisen als der Rest der Membran (2) und der Rest der Schale (1), sodass die Membran (2) ohne den Einsatz zusätzlicher Vorrichtungen an der Schale (1) haftet.

26. Verfahren zur Herstellung eines Behälters (100) gemäß einem der vorstehenden Ansprüche, das die folgenden Schritte umfasst:
A. Herstellung der Teile des genannten Behälters (100) mittels BFS durch Extrudieren eines schlauchförmigen Vorformlings aus einem geeigneten Polymer, um die Hülle (1) und die Membran (2) zu formen;
B. Montage der genannten Teile in einer geeigneten, sterilen Umgebung;
Sekundärverpackung des Behälters (100) in einer geeigneten Umgebung;
**dadurch gekennzeichnet, dass** während des Herstellungsprozesses ein Teil der Schale (1) mit einer größeren Dicke als der verbleibende Teil der Schale (1) geformt wird, um die Schale (1) steif zu machen, und ein Teil der Membran (2) mit einer geringeren Dicke als der verbleibende Teil der Membran (2) geformt wird, um die Membran (2) flexibler zu machen.

27. Herstellungsverfahren nach Anspruch 26, **dadurch gekennzeichnet, dass** während des Herstellungsprozesses und vor dem Schließen einer Form ein schalenförmiger Einsatz (101) in den Behälter eingesetzt wird, wobei der Einsatz (101) einen Aufnahmeraum (1.1) und eine Ausgabevorrichtung (1.1.1) umfasst.

28. Herstellungsverfahren nach Anspruch 25, **dadurch gekennzeichnet, dass** während der Phase der Herstellung der genannten Teile des genannten Behälters (100) bei Verwendung des Formverfahrens die folgenden weiteren Phasen stattfinden
Anordnung einer Vielzahl von Schalen (1) in einer Form unter Verwendung einer geeigneten Halterung (5.1.1) zur Anordnung der genannten Schalen (1), wobei eine heißsiegelbare Folie (5.2) an der Oberseite der herzustellenden Schalen (1) anliegt,
Erwärmung des Randes der Schale (1) und der Folie (5.2) mittels eines Verfahrens und/oder einer Kombination mehrerer Verfahren, wie beispielsweise Induktion, Heißluftblasen, Einsatz von Infrarotlampen oder Konvektion;
Druck einer Form (5.1.2) gegen die Formmatrize der noch formbaren Schalen (1), sodass die Folie (5.2) mit der Formgebung (1.6) der Schale (1) verschweißt wird; Anlegen eines Druckunterschieds an der Hülle (1) und/oder eines Drucks am Ausgabevorrichtung (1.1.1) an der Form, sodass sich die Folie (5.2) verformt und die relativ flexible Membran (2) bildet.

29. Herstellungsverfahren nach Anspruch 25 oder 28, **dadurch gekennzeichnet, dass** während des Schritts des Zusammenbaus der Teile des Behälters (100) der Ring (4) so positioniert wird, dass er Druck zwischen dem Querabschnitt (2.1) der Membran und der Formung (1.6) der Schale (1) ausübt.

30. Herstellungsverfahren gemäß den Ansprüchen 25 oder 28-29, **dadurch gekennzeichnet, dass** während des Schritts des Zusammenfügens der Teile des Behälters (100) eine Klebeschicht zwischen der Formgebung (1.6) der Schale (1) und dem Abschnitt (2.1) der Membran (2) aufgebracht wird, um die Dichtigkeit zu gewährleisten.

31. Verfahren gemäß einem der Ansprüche 25 oder 28-29, **dadurch gekennzeichnet, dass** während des Schritts des Zusammenbaus der Teile des Behälters (100) zur Erzielung einer dichten Abdichtung zwischen dem Formteil (1.6) und dem Querabschnitt (2.1) das Formteil (1.6) und der Querabschnitt (2.1) erwärmt und durch Aufbringen eines äußeren Drucks aneinandergepresst werden.

32. Herstellungsverfahren gemäß mindestens einem der Ansprüche 25 oder 28-29, **dadurch gekennzeichnet, dass** während des Schritts des Zusammenfügens der Teile des Behälters (100) Ultraschall, Infrarotstrahlung oder andere bekannte Techniken zur Förderung der mechanischen Verriegelung und des Formschlusses zwischen den Teilen verwendet werden, um den Abschnitt (2.1) der Membran (2) mit der Formgebung (1.6) der Schale (1) zu verschweißen.

33. Verfahren zur Herstellung eines Behälters (100) gemäß Anspruch 13, wie in mindestens einem der Ansprüche 25 oder 26 beschrieben, **dadurch gekennzeichnet, dass** der Behälter (100) vor der Verwendung mit der Substanz befüllt wird und das Zahnprofil der Aktivierungsvorrichtung (3) so ausgelegt ist, dass es die Membran (2) zusammendrückt, wodurch die in der Kammer (A) des Behälters (100) vorhandene Luft entfernt wird, so dass die Aktivierungsvorrichtung (3) in der ersten Betriebsposition einrastet.

## Revendications

1. Récipient (100) servant à éjecter une substance, telle que des médicaments et autres, comprenant
une coque creuse (1) destinée à contenir et à éjecter ladite substance, comprenant
une partie de confinement (1.1) comprenant un organe de distribution (1.1.1) de forme oblongue,
une membrane (2), reliée à ladite coque (1),
ledit récipient (100) comportant, dans une position contiguë à ladite partie de confinement (1.1) et transversale par rapport à celle-ci, un profilé (1.6) destiné à relier ladite membrane (2), de sorte que ladite membrane (2) puisse se déplacer depuis
une première position de fonctionnement, dans laquelle la membrane (2) présente une partie centrale située en face de la partie de confinement (1.1) de l'enveloppe (1), créant ainsi avec cette partie de confinement (1.1) une chambre (A) destinée à confiner ladite substance, pour
une deuxième position de fonctionnement, dans laquelle la partie centrale de la membrane (2) s'affaisse à l'intérieur de ladite partie (1.1) de confinement de ladite enveloppe (1), de manière à expulser ladite substance présente dans la chambre (A) par l'intermédiaire dudit organe de distribution (1.1.1) dans lequel
la membrane (2) présente des plis (F) et/ou des interstices (G) formés au cours du processus de déformation,
**caractérisé en ce que** ladite enveloppe (1) comporte des rainures (1.7), orientées vers ladite chambre (A), afin de faciliter l'éjection de ladite substance hors de ladite chambre (A),
les rainures (1.7) permettant
permettre à la substance contenue dans le récipient de s'écouler, et empêcher que celle-ci ne reste coincée dans les plis (F) et/ou les interstices (G).

2. Récipient (100) selon la revendication 1, **caractérisé en ce que** ladite membrane (2) est adaptée à la géométrie de ladite partie de confinement (1.1) de ladite enveloppe (1), de manière à adhérer à celle-ci et à être compressible dans la direction de ladite enveloppe (1), afin de projeter ladite substance contenue dans ladite chambre (A).

3. Récipient (100) selon la revendication 1 ou 2, **caractérisé en ce que** ladite membrane (2) est de type bistable, de manière à conserver de manière autonome sa propre position de fonctionnement entre lesdites positions de fonctionnement.

4. Récipient (100) selon l'une des revendications 1-3, **caractérisé en ce que** l'intensité de la force de compression exercée sur ladite membrane (2) et ladite bistabilité varient en fonction de l'épaisseur de ladite membrane (2).

5. Récipient (100) selon l'une des revendications 1-4, **caractérisé en ce que** ladite membrane (2) se présente sous la forme d'un sphéroïde allongé ou aplati, ou sous toute autre forme adaptée pour contenir ladite substance et s'affaisser à l'intérieur de l'enveloppe (1).

6. Récipient (100) selon la revendication 5, **caractérisé en ce que** ladite coque (1) comprend un ensemble d'encoches à arêtes vives (1.8) destinées à perforer ladite membrane (2) dans la deuxième position de fonctionnement, afin d'éjecter ladite substance.

7. Récipient (100) selon l'une des revendications 1-6, **caractérisé en ce que** ledit organe de distribution (1.1.1) de ladite partie de confinement (1.1) de ladite enveloppe (1) est relié à des accessoires de distribution tels que, par exemple, des aiguilles.

8. Récipient (100) selon l'une des revendications 1-7, **caractérisé en ce que** ladite coque (1) comprend une partie de support (1.5) destinée aux doigts d'un utilisateur.

9. Récipient (100) selon l'une des revendications 1-8, **caractérisé en ce qu'**il comprend un dispositif d'actionnement mobile (3), dont la forme correspond à celle de ladite coque (1), comportant une tête (3.1) adaptée pour pousser et comprimer ladite membrane (2) à l'intérieur de ladite coque (1), afin de passer de ladite première position de fonctionnement à ladite deuxième position, et un corps (3.2) adapté pour transmettre ladite compression sur ladite membrane (2) pendant l'utilisation du récipient (100).

10. Récipient (100) selon la revendication 9, **caractérisé en ce que** ladite tête (3.1) dudit dispositif d'actionnement (3) comprend une pluralité de fentes concentriques (3.6) destinées à soulager ledit dispositif (3) et à réguler la force nécessaire à la déformation de ladite membrane (2).

11. Récipient (100) selon les revendications 6 et 9, **caractérisé en ce que** ladite tête (3.1) comporte des cavités (3.8) destinées à faciliter la perforation de ladite membrane (2) au moyen desdites encoches à bords tranchants (1.8) présentes sur la surface intérieure de ladite coque (1), empêchant ainsi la réutilisation dudit récipient (100).

12. Récipient (100) selon au moins l'une des revendications 9-11, **caractérisé en ce que** ladite tête (3.1) du dispositif d'activation (3) comporte des encoches à arêtes vives aptes à perforer ladite membrane (2) après compression relative, empêchant ainsi la réutilisation dudit récipient (100).

13. Récipient (100) selon au moins l'une des revendications 9-12, **caractérisé en ce que** ledit dispositif d'activation (3) comprend un profil denté (3.7, 3.71) le long du corps (3.1), le pas entre les dents dudit profil denté (3.7, 3.71) correspondant à un volume unitaire de ladite substance à éjecter.

14. Récipient (100) selon l'une des revendications 1-13, **caractérisé en ce que** la membrane (2) et le dispositif d'activation (3) comprennent, respectivement, un raccord (2.6) et un trou (3.3) de telle sorte que ledit raccord (2.6) s'enclenche dans ledit trou (3.3) pour permettre à la membrane (2) d'être amenée dans la première position de fonctionnement et d'aspirer ladite substance dans la chambre (A).

15. Récipient (100) selon la revendication 14, **caractérisé en ce que** la membrane (2) comporte une saillie (2.3) orientée dans la direction opposée à celle du raccord (2.6), ladite saillie (2.3) étant apte à expulser la substance restant dans la partie de distribution (1.1.1).

16. Récipient (100) selon la revendication 9, **caractérisé en ce qu'**il comprend une bague (4) destinée à fixer ladite membrane (2) à ladite coque (1), ladite bague étant couplée à ladite coque (1) et étant apte à loger en son sein le dispositif d'activation (3) en guidant son mouvement, ladite coque (1) comprenant une première partie (1.2) tubulaire et creuse contiguë à ladite partie de confinement (1.1), dans laquelle ledit anneau (4) est couplé de manière à s'emboîter à l'intérieur de la première partie (1.2) de ladite enveloppe (1).

17. Récipient (100) selon l'une des revendications 1-16, **caractérisé en ce que** ladite membrane (2) comprend un bord comportant une partie transversale (2.1) se terminant par un premier sommet (2.5), contigu à ladite partie transversale (2.1) et perpendiculaire à celle-ci.

18. Récipient (100) selon la revendication 17, **caractérisé en ce que** ladite membrane (2) comprend un deuxième sommet (2.5.2), contigu et transversal à ladite partie (2.1), ledit deuxième sommet (2.5.2) étant adjacent et orienté dans la même direction que ledit sommet (2. 5), et un troisième sommet (2.5.1), perpendiculaire à la partie (2.1), situé entre les premier et deuxième sommets (2.5, 2.5.2), orienté dans la direction opposée à celle desdits premier et deuxième sommets (2.5, 2.5.2), afin d'améliorer l'étanchéité de ladite membrane (2).

19. Récipient (100) selon la revendication 9, **caractérisé en ce que** ledit dispositif d'activation (3) comprend, le long du corps (3.2), un ou plusieurs anneaux (3.4) destinés à empêcher la réutilisation du récipient (100) après usage, empêchant ainsi le retour du récipient (100) dans la première position de fonctionnement.

20. Récipient (100) selon la revendication 19, **caractérisé en ce qu'**il comprend un élément en saillie (4.2), apte à passer d'une position de repos correspondant à ladite première position de fonctionnement à une position d'activation correspondant à ladite deuxième position de fonctionnement, dans laquelle l'élément en saillie (4.2) est en prise avec au moins l'une desdites une ou plusieurs bagues (3.4) dudit dispositif d'activation (3), de manière à le fixer à la coque (1), afin d'empêcher sa réutilisation.

21. Récipient (100) selon les revendications 16 et 17, **caractérisé en ce que** ledit anneau (4) comprend un segment en saillie (4.1) dont la forme est sensiblement complémentaire de celle de ladite partie transversale (2.1) de la membrane (2), et tourné vers l'intérieur de ladite chambre (A), apte à verrouiller ladite partie transversale (2.1) de la membrane (2) au niveau du moulage (1.6) de la coque (1) en la maintenant en place et en agissant comme un joint d'étanchéité.

22. Récipient (100) selon la revendication 21, **caractérisé en ce que** ledit anneau (4) comporte, dans une position contiguë et opposée audit segment en saillie (4.1), un segment plat complémentaire (4.3) destiné à venir en contact avec ledit sommet (2.5) de ladite membrane (2).

23. Récipient (100) selon la revendication 22, **caractérisé en ce que** le segment en saillie (4.1) et ledit segment plat (4.3) de la bague (4) sont aptes à presser respectivement le sommet (2.5) et la partie transversale (2.1) de la membrane (2) à l'intérieur du moulage (1.6) de la coque (1).

24. Récipient (100) selon la revendication 21, **caractérisé en ce que** ledit anneau (4) comporte, à l'opposé du segment en saillie (4.1), un support (4.5) destiné de préférence aux doigts d'un utilisateur.

25. Procédé de fabrication d'un récipient (100) selon l'une quelconque des revendications précédentes, comprenant les étapes suivantes:
A. Fabrication des éléments dudit récipient (100) par moulage par injection et/ou par compression, lesdits éléments comprenant la coque (1) et la membrane (2);
B. Assemblage desdites pièces dans un environnement stérile adapté;
Emballage secondaire du conteneur (100) dans un environnement approprié;
C. Stérilisation finale du conteneur (100) à l'aide d'une ou plusieurs méthodes de stérilisation connues **caractérisé en ce que**, au cours du processus de fabrication, une étape consiste à contrôler le processus de refroidissement desdites pièces de manière à ce que la partie profilée (1.6) et la partie transversale (2.1) présentent une température plus élevée que le reste de la membrane (2) et le reste de la coque (1), afin que la membrane (2) adhère à la coque (1) sans recourir à des moyens supplémentaires.

26. Procédé de fabrication d'un récipient (100) selon l'une quelconque des revendications précédentes, comprenant les étapes suivantes:
A. Fabrication des éléments dudit conteneur (100) par BFS, par extrusion d'une ébauche tubulaire en polymère approprié afin de mouler l'enveloppe (1) et la membrane (2);
B. Assemblage desdites pièces dans un environnement stérile adapté;
Emballage secondaire du conteneur (100) dans un environnement approprié;
**caractérisé en ce que**, au cours du processus de fabrication, une partie de la coque (1) est moulée avec une épaisseur supérieure à celle du reste de la coque (1) afin de rendre la coque (1) rigide, et une partie de la membrane (2) est moulée avec une épaisseur inférieure à celle du reste de la membrane (2) afin de rendre ladite membrane (2) plus souple.

27. Procédé de fabrication selon la revendication 26, **caractérisé en ce que**, au cours du processus de fabrication et avant la fermeture d'un moule, un insert en forme de coque (101) est ajouté audit récipient, ledit insert (101) comprenant une partie de confinement (1.1) et un organe de distribution (1.1.1).

28. Procédé de fabrication selon la revendication 25, **caractérisé en ce que**, au cours de la phase de réalisation desdites parties dudit récipient (100), lorsque la technique de moulage est utilisée, les phases supplémentaires suivantes sont prévues Disposition d'une pluralité de coques (1) à l'intérieur d'un moule à l'aide d'un gabarit (5.1.1) adapté à la disposition desdites coques (1), dans lequel un film thermosoudable (5.2) est placé contre la face supérieure des coques (1) en cours de fabrication,
Chauffage du bord de la coque (1) et du film (5.2) à l'aide d'une méthode et/ou d'une combinaison de plusieurs méthodes, telles que, par exemple, l'induction, le soufflage d'air chaud, l'utilisation de lampes à infrarouge ou la convection;
Pression exercée par un moule (5.1.2) contre la matrice de moulage des coques (1) encore malléables, de manière à souder le film (5.2) au profil (1.6) de la coque (1);
Application d'une différence de pression au niveau de l'enveloppe (1) et/ou d'une pression au niveau de l'organe de refoulement (1.1.1) sur la matrice de moulage, de telle sorte que le film (5.2) se déforme pour former la membrane relativement souple (2).

29. Procédé de fabrication selon la revendication 25 ou 28, **caractérisé en ce que**, au cours de l'étape d'assemblage des pièces dudit récipient (100), l'anneau (4) est positionné de manière à exercer une pression entre la partie transversale (2.1) de la membrane et le moulage (1.6) de la coque (1).

30. Procédé de fabrication selon les revendications 25 ou 28-29, **caractérisé en ce que**, au cours de l'étape d'assemblage desdites pièces du récipient (100), une couche de colle est appliquée entre le profilé (1.6) de la coque (1) et la partie (2.1) de la membrane (2), afin d'assurer l'étanchéité.

31. Procédé de fabrication selon l'une des revendications 25 ou 28-29, **caractérisé en ce que**, au cours de l'étape d'assemblage desdites parties du récipient (100), afin d'obtenir une étanchéité parfaite entre la partie profilée (1.6) et la partie transversale (2.1), ladite partie profilée (1.6) et ladite partie transversale (2.1) sont chauffées et pressées l'une contre l'autre par l'application d'une pression externe.

32. Procédé de fabrication selon au moins l'une des revendications 25 ou 28-29, **caractérisé en ce que**, au cours de l'étape d'assemblage desdites pièces dudit récipient (100), on utilise des ultrasons, des infrarouges ou d'autres techniques connues pour favoriser l'engagement mécanique et l'interférence entre lesdites pièces afin de souder la partie (2.1) de la membrane (2) à la moulure (1.6) de la coque (1).

33. Procédé de fabrication d'un récipient (100) selon la revendication 13, tel que défini au moins dans l'une des revendications 25 ou 26, **caractérisé en ce que**, avant utilisation, ledit récipient (100) est rempli de ladite substance et ledit profil denté dudit dispositif d'activation (3) est conçu pour comprimer ladite membrane (2), éliminant l'air présent dans ladite chambre (A) dudit récipient (100), de sorte que ledit dispositif d'activation (3) se verrouille dans ladite première position de fonctionnement.
